# EUROPEAN PATENT APPLICATION

(11) **EP 4 170 048 A1**
(43) Date of publication of application: **26.04.2023**
(21) Application number: 22150455.8
(22) Date of filing: 06.01.2022
(51) Int. Cl.: C12Q 1/6883

(54) **EVALUATION SYSTEM FOR EVALUATING PSORIASIS AND USE THEREOF**

(30) Priority: 25.10.2021 CN 202111239725
(71) Applicant: Sun, Liangdan, Hefei City, Anhui 230022 (CN)
(72) Inventor: SUN, Liangdan, Hefei City, Anhui 230022 (CN); ZHEN, Qi, Hefei City, 230022 (CN); LI, Bao, Hefei City, 230022 (CN)
(74) Representative: Ipside

(57) **Abstract**

The present invention belongs to the field of medical information technology, and specifically relates to an evaluation system for evaluating psoriasis and use thereof. The evaluation system comprises: a data acquisition module for acquiring the dsDNA content of a serum sample to be detected; a data analysis module for evaluating psoriasis based on the dsDNA content; a data output module for outputting results according to the evaluated psoriasis, wherein if the serum sample to be detected is a serum sample without phenotype and the dsDNA content is ≥ 1.11 ng/ml, the output would be: abnormal and extremely high risk. The sensitivity of the evaluation system is 61.6% and the specificity is 74.8%. Compared with other types of psoriasis-related methods and apparatuses, the sensitivity and specificity have obvious advantages.

## Description

### Technical Field

The present invention belongs to the field of medical information technology, and specifically relates to an evaluation system for evaluating psoriasis and use thereof.

### Background Art

Psoriasis is a seasonal, recurrent, latitude-related autoimmune disease, and its pathogenesis has not yet been elucidated. Clinical observation and studies have found that psoriasis is more likely to be induced, recur or be aggravated in winter, and has an epidemiological feature that the prevalence in high latitude areas is higher than that in low latitude areas. The underlying reasons that cause the prevalence of psoriasis to vary with seasons and latitudes are still a mystery. Studies have shown that geographical and climatic factors, comprising latitudes and seasons, may affect the development of the disease by affecting immunogenic substances. dsDNA, as an innate immune stimulant commonly associated with a variety of immune diseases, affects the occurrence and development of many immune diseases. Exploring the serum dsDNA of patients with psoriasis in different seasons and latitudes has a breakthrough effect in revealing why psoriasis has seasonal and latitude-related changes.

Serum free double-stranded DNA (dsDNA), as a stimulant of innate immunity, is one of the important factors closely related to immune diseases, has a high degree of immunogenicity, and can be recognized by a receptor to further trigger an innate immune response, thereby activating the immune system and ultimately leading to the occurrence of autoimmune diseases. DsDNA has been reported to be related to many immune inflammatory diseases comprising atopic dermatitis and rheumatoid arthritis, and most of these diseases have a potential latitudinal or seasonal aggravation trend in epidemiology. Climate would affect the physiological state of immunity and even the progression of diseases. Studies have shown that immune-related gene transcriptome in European populations increases in winter, and pro-inflammatory factors, which are biomarkers of autoimmune disease risk, also peak in winter. These phenomena imply that climatic factors, comprising latitudes and seasons, may affect the development of the disease by affecting immunogenic substances.

There is currently no research on high-throughput detection of serum free dsDNA, and it is impossible to systematically describe the basic level of serum free dsDNA in healthy people. There is no definite standard for the normal range of serum free dsDNA in healthy people. Most of the international studies on serum dsDNA are qualitative studies, and the application areas are mainly focused on non-invasive prenatal screening, especially the sequencing for tumors. A small number of studies use detection methods such as spectrophotometers to quantitatively study dsDNA, but such methods cannot be used for large-scale detection and have limitations in batch detection clinically. Both for healthy people and for people with psoriasis, till now there is no low-cost and accurate method to detect serum dsDNA, and it is unable to carry out quantitative analysis on large sample size, so it is difficult to perform the correlation analysis between psoriasis and serum dsDNA. Moreover, there is no research on elucidating the distribution pattern of serum dsDNA in the normal population, and no studies have made a specific analysis on the correlation between psoriasis and serum dsDNA, thus the causal relationship between psoriasis and serum dsDNA and the epidemiological rules contained in the causal relationship are still not clear.

### Summary of the Invention

The purpose of the present invention is to provide an evaluation system for evaluating psoriasis, and the evaluation system can predict the phenotype of serum sample without phenotyping and phenotypic changes in phenotyped serum sample.

The evaluation system comprises:
a data acquisition module for acquiring the dsDNA content of a serum sample to be detected;
a data analysis module for evaluating psoriasis based on the dsDNA content;
a data output module for outputting results according to the evaluated psoriasis, wherein
if the serum sample to be detected is a serum sample without phenotype and the dsDNA content is ≥1.11 ng/ml, the output would be: abnormal and extremely high risk.

Specifically, dsDNA is an immune stimulant commonly associated with a variety of immune diseases, it is extremely important to determine the distribution range of serum free dsDNA and the tendency of serum free dsDNA that varies with seasons and latitudes in the serum of healthy people to guide the diagnosis and treatment of dsDNA-related diseases, and serum free dsDNA may be a clinical indicator for assessing disease risk and prognosis.

Specifically, from the analysis of the characteristic work curve of a large number of samples, it is found that the optimal cut-off value of serum dsDNA for diagnosing psoriasis is 1.11 ng/ml, the sensitivity is 61.6%, and the specificity is 74.8%. In a multi-factor regression prediction model, the introduction of serum dsDNA can significantly increase the area under the curve used for predicting psoriasis from 0.806 to 0.860 (P < 0.001). When the optimal cut-off value of dsDNA is used as a reference, there is a significant dose-response relationship between the serum dsDNA level and the risk of occurrence of psoriasis.

Further, if the serum sample to be detected is a serum sample without phenotype and the dsDNA content is 0.97 ng/ml ≤ dsDNA content < 1.11 ng/ml, the output would be: normal and high risk; if the dsDNA content is 0.86 ng/ml ≤ dsDNA content < 0.97 ng/ml, the output would be: normal; or if the dsDNA content is 0.86 ng/ml, the output would be: suspicious of other diseases.

Specifically, the present invention presents the fluctuating range of the basic level of serum dsDNA in healthy Chinese people for the first time. The median level of serum dsDNA in normal people is 0.97 ng/ml (interquartile range: 0.86-1.11 ng/ml). At the same time, if the serum dsDNA level in healthy people is between 0.97 to 1.11 ng/ml, the risk of psoriasis is relatively high and can be determined at high-risk.

Further, in order to better monitor whether there is a risk of psoriasis and the degree of risk for potential patients or normal people (that is, the serum sample to be detected are serum sample without phenotype), serum sample can be obtained at different time points to detect and record dsDNA content. In the case that the serum sample to be detected is a serum sample without phenotype, the data acquisition module acquires the dsDNA content twice or more at different time points, for every one standard deviation increase in the dsDNA content at the later time point from the dsDNA content at the previous time point, the risk increases by 1.84 times (OR: 2.84, 95% CI: 2.01-4.01), and the output module outputs whether the dsDNA content is normal and the corresponding risk factor.

Specifically, long-term monitoring of serum dsDNA in normal people can prompt the people to control personal behaviors that increase dsDNA (such as smoking, alcohol use, infection, frostbite, trauma, use of cytotoxic drugs, *etc.*) to keep serum dsDNA low, thereby reducing the morbidity. Long-term monitoring of serum dsDNA in high-risk people can predict the dynamic risk of psoriasis and the severity of psoriasis in the high-risk people. Serum dsDNA can be used as a warning value to remind patients when necessary clinical interventions are needed. For patients who have been treated, dynamic monitoring of serum dsDNA can dynamically indicate the effect of treatment. If the serum dsDNA content exceeds 0.97 ng/ml after treatment, it may indicate that the patients still have a higher risk of relapse and an imperfect prognosis. For people with low dsDNA content whose serum dsDNA content is less than 0.86 ng/ml, they are at risk of suffering from other diseases caused by dsDNA expression. Dynamic monitoring of changes in dsDNA content can warn the people for further diagnosis and treatment for the other diseases of dsDNA.

Further, in the case that the serum sample to be detected is a serum sample without phenotype, when the dsDNA content is < 1.11 ng/ml, for every 0.12 ng/ml increase in the difference between the dsDNA content at the later time point and the dsDNA content at the previous time point recorded by the data acquisition module, the OR value of the risk of occurrence of psoriasis is 4.88 (95% CI: 3.85-6.20), and the output module outputs whether the dsDNA content is normal and the corresponding OR value; when the dsDNA content is ≥1.11 ng/ml, for every 0.34 ng/ml increase in the difference between the dsDNA content at the later time point and the dsDNA content at the previous time point recorded by the data acquisition module, the OR value of the risk of occurrence of psoriasis is 1.97 (95% CI: 1.74-2.22), and the output module outputs whether the dsDNA content is normal and the corresponding OR value.

Specifically, the present invention performs serum dsDNA detection on a large number of samples from people with psoriasis and healthy people. After standardization, a multicenter statistical analysis is performed to determine the direction and strength of correlation between psoriasis and serum dsDNA in terms of severity score, patient's living habits, and the climatic conditions under which patients live. The correlation between serum dsDNA and psoriasis has been verified from many aspects. At the same time, according to the epidemiological statistical results of a large number of samples from people, the distribution range of serum dsDNA of psoriasis patients and the critical value of serum dsDNA for the diagnosis of psoriasis have been determined for the first time, which has important scientific value for the diagnosis and prediction of psoriasis. The results of the study show that the serum dsDNA of patients with psoriasis is significantly higher than that of normal people, and consistent with the rule that the serum dsDNA level of patients in high latitude areas is significantly higher than that of patients in low latitude areas, and the serum dsDNA level of patients in winter is significantly higher than the serum dsDNA level of patients in summer. The results of risk correlation analysis show that for every 1 ng/ml increase in serum dsDNA, the risk of occurrence of psoriasis increases by 40.40 times (adjusted odds ratio, 41.40; 95% confidence interval, 32.32-53.03, *P*< 0.001).

Further, in the case that the serum sample to be detected is a phenotyped and cured serum sample, and the dsDNA content is ≥1.11 ng/m, then the output would be: very likely to relapse. If the serum dsDNA level is still higher than 1.11 ng/ml after treatment, there is a high probability of relapes of the disease, which is of great significance for prognostic risk assessment. Therefore, regular serum dsDNA detection can be performed on cured patients without psoriasis lesions to monitor the relapse of psoriasis in the patients.

Further, in the case that the serum sample to be detected is a phenotyped serum sample, the data acquisition module acquires the dsDNA content twice or more at different time points, the data analysis module uses a PASI method for evaluation, and the data output module outputs corresponding results; the PASI method is one of the following: (1) for the phenotyped serum sample, for every 1 ng/ml increase in the dsDNA content at the later time point from the dsDNA content at the previous time point, the risk of being rated as moderate by PASI-1 increases by 0.66 times (OR: 1.66, 95% CI: 1.10-2.50), and the risk of being rated as severe by PASI-1 increases by 1.43 times (OR: 2.43, 95% CI: 1.77-3.35); or (2) for the phenotyped serum sample, for every 1 ng/ml increase in the dsDNA content at the later time point from the dsDNA content at the previous time point, the risk of being rated as moderate by PASI-2 increases by 0.78 times (OR: 1.78, 95% CI: 1.29-2.46), and the risk of being rated as severe by PASI-2 increases by 1.38 times (OR: 2.38, 95% CI: 1.59-3.57).

Further, the present invention also proves that the serum dsDNA level is significantly positively correlated with the severity of psoriasis.

Further, in the case that the serum sample to be detected is a phenotyped serum sample, the data acquisition module acquires the dsDNA content twice or more at different time points, the data analysis module uses a BSA method for evaluation, and the data output module outputs corresponding results; the BSA method is one of the following: (1) for the phenotyped serum sample, for every 1 ng/ml increase in the dsDNA content at the later time point from the dsDNA content at the previous time point, the risk of being rated as moderate by BSA-1 increases by 0.83 times (OR: 1.83, 95% CI: 1.12-2.97), and the risk of being rated as severe by BSA-1 increases by 1.87 times (OR: 2.87, 95% CI: 1.90-4.33); or (2) for the phenotyped serum sample, for every 1 ng/ml increase in the dsDNA content at the later time point from the dsDNA content at the previous time point, the risk of being rated as moderate by BSA-2 increases by 0.66 times (OR: 1.66, 95% CI: 1.13-2.45), and the risk of being rated as severe by BSA-2 increases by 1.61 times (OR: 2.61, 95% CI: 1.84-3.72).

Further, the evaluation system further comprises a case module for recording the dsDNA content of the serum sample to be detected at different time points and whether there is a history of psoriasis.

Further, the method for detecting dsDNA content is selected from a method such a spectrophotometer method or a double-stranded DNA quantitative detection method.

In some specific embodiments, the method for detecting serum dsDNA content adopts double-stranded DNA quantitative detection which specifically comprises:
(1) serum collection: fresh venous whole blood is collected using a vacuum coagulation-promoting tube and placed at room temperature for 1 hour, and then centrifuged in an eppendorf centrifuge at 4°C and 4000 rpm for 30 minutes, and then the supernatant is taken.
(2) serum storage: for short-term storage, the supernatant can be transferred to EP tube and stored in the refrigerator at -80°C; for long-term storage, the supernatant can be stored in a cryotube in liquid nitrogen.
(3) detection (refer to the Biovision EZQuant dsDNA operating instruction) preparation: after the serum sample has been collected, the detection would be carried out. 1x TE buffer, 2x dsDNA staining solution and 10 ng/µl of standard are prepared according to the instruction in the kit, respectively.
(4) standard curve preparation: the linear standard is prepared by formulating 10 ng/µl of standard according to the Biovision EZQuant dsDNA instruction. 0, 2, 4, 6, 8 and 10 µl of standard sample is added to corresponding well of the black microtiter plate, respectively. The standard sample is made up to 50 µl with 1x TE buffer. Three replicates are set for each concentration.
(5) sample preparation: 10 µl of the sample is added to each well of the black microtiter plate and then 1x TE buffer is used to make up to 50 µl. Three replicates are set for each concentration.
(6) staining: 50 µl of 2x dsDNA staining solution is added to each well, the black microtiter plate is placed on a shaker and gently shaken at room temperature for 5 minutes away from light.
(7) detection: after the staining reaction is completed, the microplate reader with Ex/Em = 480/530 detection module or similar apparatus is used for immediate detection.
(8) calibration: each plate with wells should be designed with a standard curve and a 0 hole. After the detection results come out, the standard curve and the 0 hole is firstly used for calibration. Generally, if the r2 value of the linear correlation relationship of the standard curve should be greater than 0.99, the detection result is good, and the data can be used.

The purpose of the present invention is to also provide the use of a dsDNA binding agent in the preparation of a psoriasis detection reagent/kit. The dsDNA binding agent is a substance that can chemically or biologically bind to dsDNA to produce changes.

The purpose of the present invention is to provide an apparatus for the treatment of psoriasis, which comprises an ultraviolet emitting device.

In embodiments of the present invention, people who is male, over 40 years old, in winter, in cities at northern region or mid-latitude region, in cities with low temperature, low ultraviolet level, low humidity, low sunlight intensity or short sunlight duration have significantly higher dsDNA level. Among all climatic factors, ultraviolet rays have the greatest impact on the fluctuation of serum dsDNA. It is possible to treat dsDNA-related autoimmune diseases through ultraviolet intervention therapy. At the same time, it is suggested that healthy people living in high latitude and low latitude areas should receive adequate ultraviolet radiation to achieve the purpose of preventing psoriasis and other related autoimmune diseases.

The present invention explains the reasons for the aggravation of psoriasis caused by some lifestyle habits and the effectiveness of certain clinical treatments: in the clinical treatment of psoriasis, the behaviors that can increase serum dsDNA, such as smoking, alcohol use, skin damage, *etc.,* can induce or aggravate psoriasis, and the behaviors that can reduce serum dsDNA, such as moisturizing and receiving ultraviolet radiation, can treat psoriasis.

The purpose of the present invention is to also provide a high-throughput detection and analysis method for dsDNA. Prior to this, the detection of serum dsDNA basically used a spectrophotometer, which could not meet the needs of clinical detection to conduct batch detection of samples.

The high-throughput detection and analysis method comprises:
(1) detecting serum dsDNA content by using a double-stranded DNA quantitative detection method;
(2) calibrating the detection results with a standard curve and a 0 hole, and the data with the r2 value of the linear correlation relationship of the standard curve greater than 0.99 being retained;
(3) performing data processing and analysis by using SPSS and R Version.

Further, the method also comprises the steps such as serum collection and serum preservation for later use.

In some specific embodiments, the detection and analysis method comprises:
(1) serum collection: fresh venous whole blood is collected using a vacuum coagulation-promoting tube and placed at room temperature for 1 hour, and then centrifuged in an eppendorf centrifuge at 4°C and 4000 rpm for 30 minutes, and then the supernatant is taken.
(2) serum storage: for short-term storage, the supernatant can be transferred to EP tube and stored in the refrigerator at -80°C; for long-term storage, the supernatant can be stored in a cryotube in liquid nitrogen.
(3) detection (refer to the Biovision EZQuant dsDNA operating instruction) preparation: after the serum sample has been collected, the detection would be carried out. 1x TE buffer, 2x dsDNA staining solution and 10 ng/µl of standard are prepared according to the instruction in the kit, respectively.
(4) standard curve preparation: the linear standard is prepared by formulating 10 ng/µl of standard according to the Biovision EZQuant dsDNA instruction. 0, 2, 4, 6, 8 and 10 µl of standard sample is added to corresponding well of the black microtiter plate, respectively. The standard sample is made up to 50 µl with 1x TE buffer. Three replicates are set for each concentration.
(5) sample preparation: 10 µl of the sample is added to each well of the black microtiter plate and then 1x TE buffer is used to make up to 50 µl. Three replicates are set for each concentration.
(6) staining: 50 µl of 2x dsDNA staining solution is added to each well, the black microtiter plate is placed on a shaker and gently shaken at room temperature for 5 minutes away from light.
(7) detection: after the staining reaction is completed, the microplate reader with Ex/Em = 480/530 detection module or similar apparatus is used for immediate detection.
(8) calibration: each plate with wells should be designed with a standard curve and a 0 hole. After the detection results come out, the standard curve and the 0 hole is firstly used for calibration. Generally, if the r2 value of the linear correlation relationship of the standard curve should be greater than 0.99, the detection result is good, and the data can be used.
(9) statistical analysis of data: SPSS 23.0 and R Version 4.0.2 software are used for data processing and analysis. All tests use two-sided tests, *P* value less than 0.05 is considered as statistically significant.

In the present invention, the expression "serum sample without phenotype" refers to a serum sample from people who has not shown clinical symptoms on the skin; "phenotyped cured serum sample" refers to a serum sample from people who has had clinical symptoms on the skin but have been cured; "serum sample with phenotype" refers to a serum sample from people who have shown clinical symptoms.

In the present invention, the expression "extremely high risk" refers that the content of dsDNA has exceeded the normal level, but there is no phenotype yet, and the phenotype may be about to emerge. "Normal and high-risk" refers that the dsDNA content is normal, but there is a tendency to evolve into psoriasis, just like the case of high-segment glycemic index in normal blood sugar.

### The beneficial effects of the present invention

The sensitivity of the evaluation system for evaluating psoriasis provided by the present invention is 61.6% and the specificity is 74.8%. Compared with other types of psoriasis-related methods and apparatus, the sensitivity and specificity have obvious advantages.

Compared with other detection methods, the high-throughput detection method for free dsDNA in the circulatory system provided by the present invention has higher detection sensitivity, better accuracy, higher result stability, shorter detection period, higher detection efficiency, and lower detection cost, and is convenient, affordable and simple, and more suitable for clinical large-scale and large-batch detection applications.

### Brief Description of the Drawings

Figure 1 shows the experimental design flow chart.
Figure 2 shows the distribution of sample sources.
Figure 3 shows the standard curve detected by biotek cell vision 5.
Figure 4 shows the standard curve detected by flex station 3.
Figure 5 shows the comparison of dsDNA levels between patients with psoriasis and the control group.
Figure 6 shows the comparison of dsDNA levels between patients with psoriasis and the control group after stratification.
Figure 7 shows the comparison of serum dsDNA levels in the control groups in different classification subgroups.
Figure 8 shows a hypothetical model of the correlation between serum dsDNA and psoriasis.
Figure 9 shows the ROC curve for the serum dsDNA to predict psoriasis.
Figure 10 shows the ROC curve for the serum dsDNA to predict psoriasis in a multivariate logistic regression model.
Figure 11 shows the restricted cubic spline curve relationship between the dsDNA level and the risk of occurrence of psoriasis.
Figure 12 shows the relationship between dsDNA and psoriasis risk in different regions (a: south China, b: north China).
Figure 13 shows the relationship between dsDNA and psoriasis risk in different latitudes (a: low latitude, b: high latitude).
Figure 14 shows the relationship between dsDNA and the risk of occurrence of psoriasis at different temperatures (a: low temperature, b: high temperature).
Figure 15 shows the relationship between dsDNA and psoriasis risk in different seasons (a: spring and autumn, b: summer, c: winter).
Figure 16 shows the relationship between dsDNA and psoriasis risk at different ultraviolet degrees (a: low and medium ultraviolet degrees, b: high ultraviolet degrees).
Figure 17 shows the relationship between dsDNA and psoriasis risk at different daylight durations (a: short daylight duration, b: medium daylight duration, c: long daylight duration).
Figure 18 shows the relationship between dsDNA and psoriasis risk at different intensities of sunlight exposure.
Figure 19 shows the relationship between serum dsDNA content and psoriasis risk without adjusting for confounding factors.
Figure 20 shows the correlation analysis between the serum dsDNA level and the grade of psoriasis.

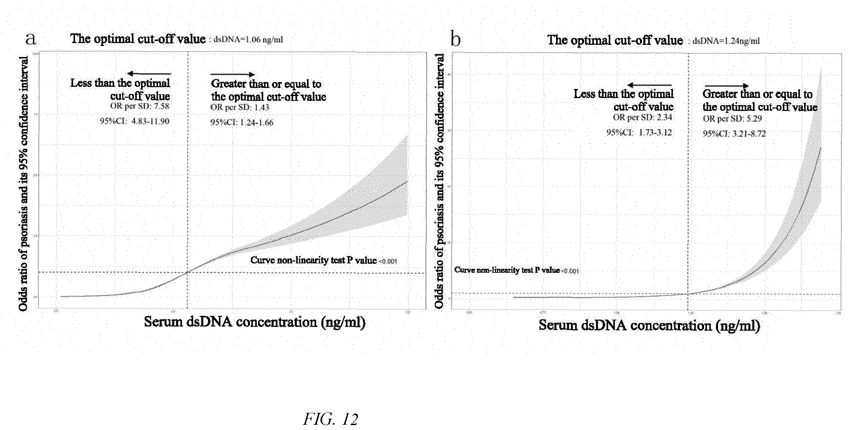

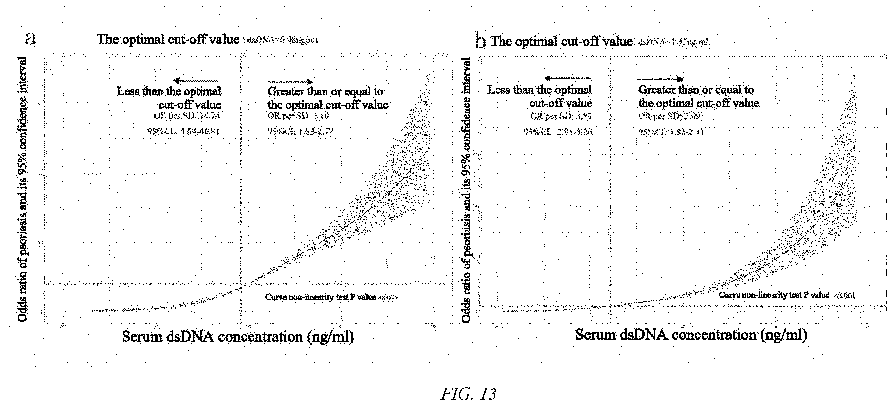

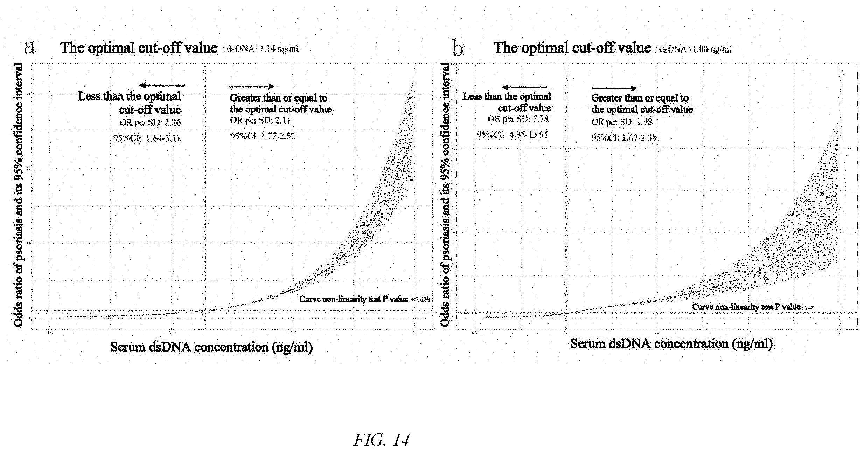

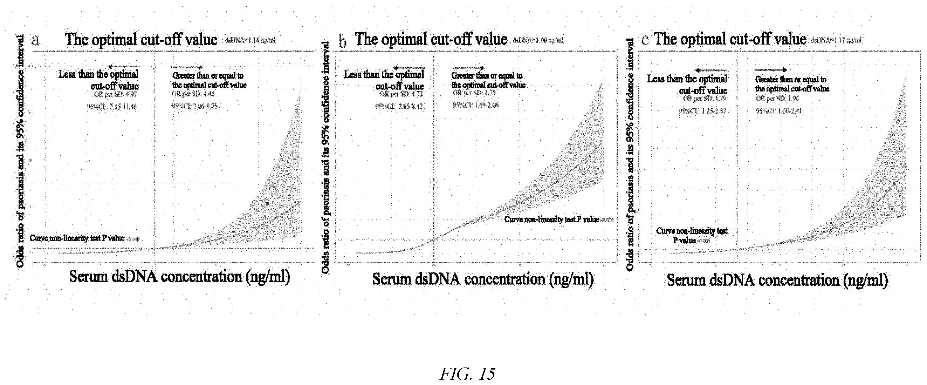

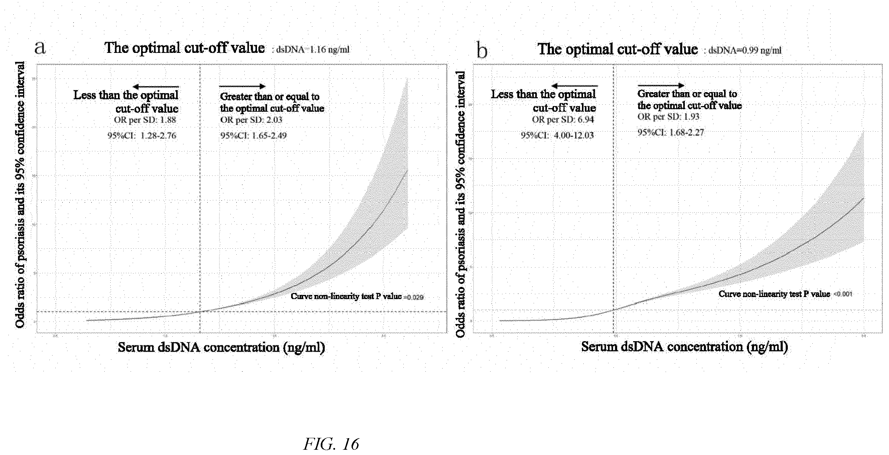

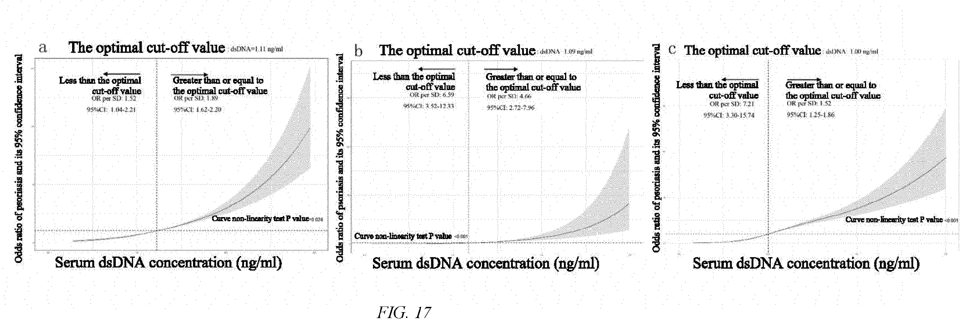

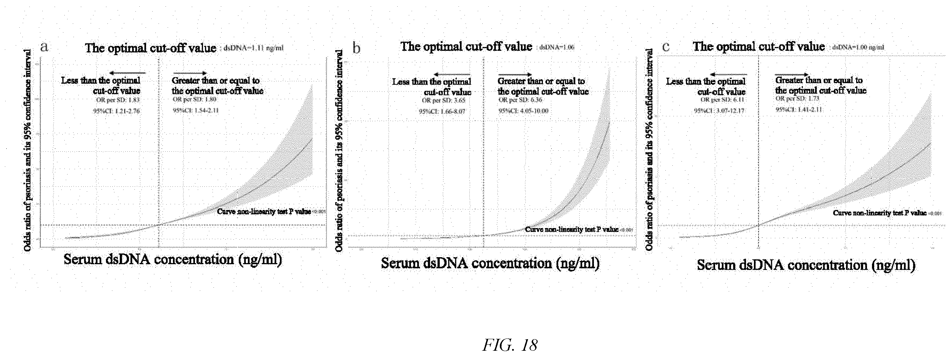

Among these figures, in Figure 12-18, the ROC curve of dsDNA is used to determine the reference value of dsDNA value, which is used to predict the psoriasis of all subjects in different regions. Adjustment is performed according to age, gender, body mass index, season, latitude, temperature, ultraviolet ray, humidity, daylight duration and sunlight exposure duration.

### Detailed Description of Embodiments

The examples given are to better illustrate the present invention, but the content of the present invention is not limited to the examples given. Therefore, the non-essential improvements and adjustments made by those skilled in the art to the examples based on the above-mentioned content of the invention still belong to the protection scope of the present invention.

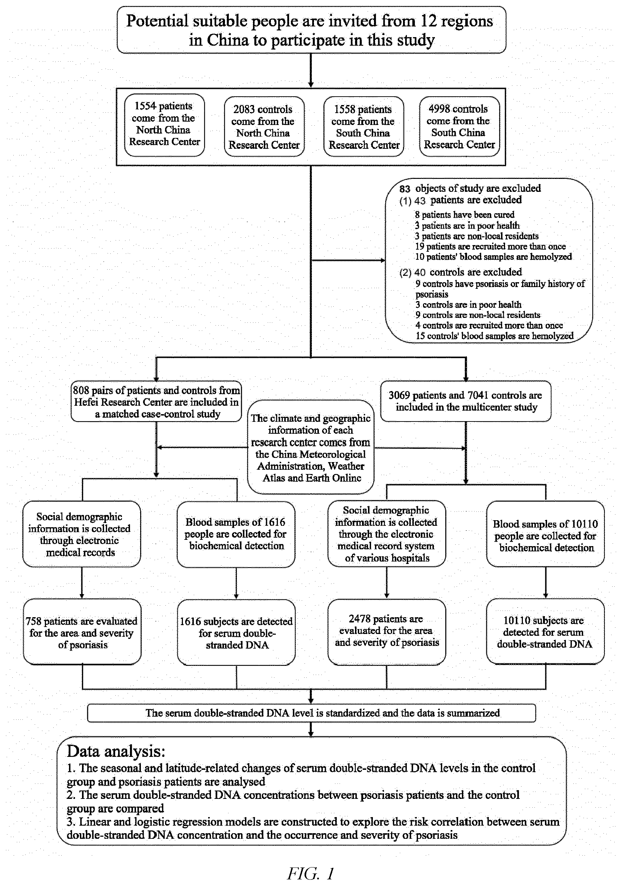

In examples of the present invention, test verification is carried out according to the process shown in Figure 1.

In examples of the present invention, the consumables needed to carry out this detection are: vacuum coagulation-promoting tube (BD Vacutainer, product number: 367957, USA), enzyme-free EP tube, cryotube, black 96-well culture plate (costar^{®}, 2 Alfred Rd, Kennebunk ME 04043, USA), Biovision EZQuant dsDNA fluorescence detection kit (Ex/Em = 480/530), nuclease-free water.

In examples of the present invention, the apparatuses needed to carry out this detection are: a centrifuge (Eppendorf, model: 581 0R, Germany) with a 15 ml adapter, a microplate reader capable of Ex/Em = 480 /530 and a shaker.

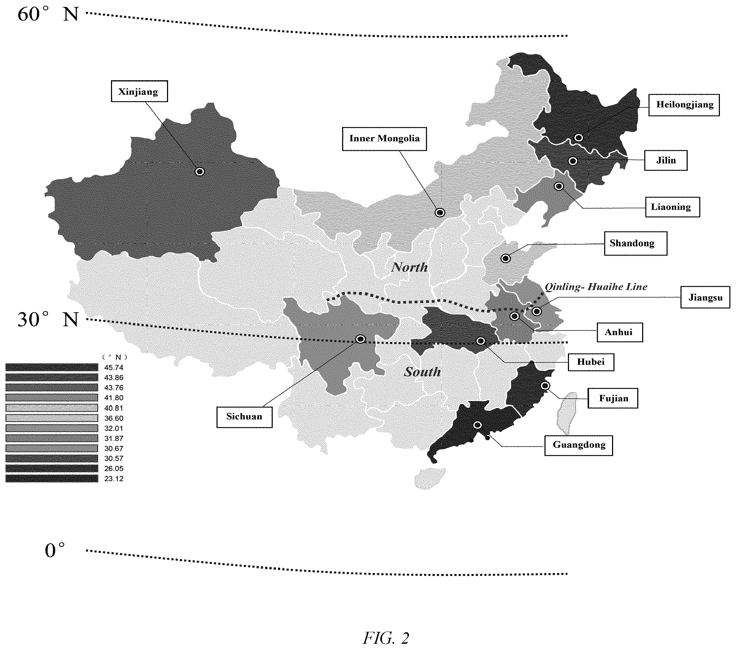

The sample selection for this detection in embodiments of the present invention (the distribution of sample sources is shown in Figure 2) is that: people with psoriasis and healthy people are recruited in 12 regions at different latitudes (south/latitude ≤ 30°; north/latitude > 30°) in winter (October-March) and summer (April-September) in China from 2016 to 2020 (the patients are from Heilongjiang, Jilin, Liaoning, Xinjiang, Inner Mongolia, Shandong, Anhui, Jiangsu, Hubei, Sichuan and Guangdong provinces; the healthy controls are from Heilongjiang, Liaoning, Inner Mongolia, Anhui, Guangdong and Fujian provinces).

In examples of the present invention, all healthy control people are enrolled from the medical examination center of the hospital medical center, and are comprised in the study according to the following inclusion criteria: (1) voluntary participation and signed informed consent; (2) no restrictions on age and gender; (3) only one control is recruited in a family unit; (4) the control people is a permanent resident of the area where the medical center is located and has no long-term travel history or residence history in other places outside the area. Controls that meet the following exclusion criteria are excluded: (1) people diagnosed with psoriasis, autoimmune diseases or systemic diseases, and people having any reported family history of psoriasis (comprising first-degree, second-degree and third-degree relatives); (2) the control people is not a permanent resident of the area where the medical center is located; (3) poor physical health caused by serious diseases, such as tumors, coagulation dysfunction, severe anemia, involuntary consciousness, *etc.;* (4) volunteers have been comprised in the study more than once; (5) the blood of volunteers is hemolyzed before the experiment.

In examples of the present invention, each participant has signed a written informed consent. For participants under the age of 18 or over 65, the informed consent of their guardian is also needed to obtain. The trained field technicians use a unified standardized questionnaire to interview the subjects. All people with psoriasis are diagnosed with psoriasis by two senior dermatologists. Patients who are cured and have no skin damage and patients with other serious diseases that seriously affect their health are excluded. A total of 3069 psoriasis patients and 7041 healthy people are comprised for the detection of serum dsDNA levels. A total of 10110 subjects are enrolled, with an average age of 43.03 ± 15.74 years old. Among all participants, 5,640 are men, accounting for 55.8%. All enrolled patients and healthy people give informed consent and volunteer to collect 1 mL blood samples. The study is approved by the institutional ethics committee and is conducted in accordance with the principles of the Declaration of Helsinki. When evaluating the results, the researchers are unaware of the group assignments during the experiment.

In examples of the present invention, the sample information collected for this detection is:
(1) psoriasis patients: data on relevant demographic characteristics are collected at admission to hospital; the dermatologist record and use the PASI score and BSA score to evaluate the patient's condition; in addition, the patient's history of other diseases, smoking status, alcohol status, time of admission to hospital, and blood pressure, blood lipids, blood sugar, liver function and other clinical laboratory indicators at admission to hospital are also collected. (2) healthy control people: the basic demographic information such as age and gender of healthy people are obtained from the hospital physical examination center; in addition, information on blood pressure, blood lipids, blood sugar, liver function and other clinical laboratory indicators are also collected during physical examination.

In examples of the present invention, the variables used to carry out this detection are: the variables in the experiment are the monthly average temperature, ultraviolet (UV) index, relative humidity, sunlight exposure duration and daylight duration, *etc.* for 12 regions collected by China Meteorological Administration, Weather Atlas and Earth Online.
(1) According to the latitude classification defined by Weather Atlas, 0° to 30° (not comprising 30°) refers to low latitude, and 30° to 60° (not comprising 60°) refers to mid-latitude.
(2) According to the geographical division of China, it is generally believed that the Qinhuai Line divides China into the northern region and the southern region.
(3) The monthly average ultraviolet index for 6 regions is obtained from Weather Atlas. The ultraviolet index is divided into low (less than 2), medium (3 to 5), high (6 and 7), very high (8 to 10), and extreme (11 and above).
(4) Relative humidity refers to the moisture content in the air, which is queried from Weather Atlas and quantified according to the appropriate humidity for healthy housing. The standard is 40-70% relative humidity. Relative humidity < 70% and> 70% are divided into low humidity and high humidity, respectively.
(5) Daylight duration refers to the amount of time from sunrise to sunset, while sunlight exposure duration refers to how much bright sunlight can be received. Daylight duration and sunlight exposure duration are sorted by statistical quartile. Daylight duration is divided into 8.7-11.1 hours, 11.2-13.3 hours and 13.4-15.7 hours, which are defined as short daylight duration, medium daylight duration and long daylight duration, respectively. The sunlight exposure duration is divided into 2-5.83 hours, 5.84-7.70 hours and 7.71-11.8 hours, which are defined as short sunlight exposure duration, medium sunlight exposure duration and long sunlight exposure duration, respectively.
(6) The severity of psoriasis is classified according to PASI and BSA. In order to ensure the accuracy of the assessment, four recognized classification methods are used to classify the severity of psoriasis. PASI scores of < 8, 8-12 and> 12 are determined as mild, moderate or severe psoriasis by the first assessment method, which is defined as PASI-1. PASI scores of < 10, 10-20 and> 20 are determined as mild, moderate or severe psoriasis by the second assessment method, which is defined as PASI-2. BSA scores of < 5, 5-10, and> 10 represent the third method to define mild, moderate or severe psoriasis and the method is defined as BSA-1. BSA scores of < 10, 10-20, and> 20 represent the fourth method to define mild, moderate or severe psoriasis and the method is defined as BSA-2.

In examples of the present invention, the specific steps for carrying out this detection are:
(1) serum collection: fresh venous whole blood is collected using a vacuum coagulation-promoting tube and placed at room temperature for 1 hour, and then centrifuged in an eppendorf centrifuge at 4°C and 4000 rpm for 30 minutes, and then the supernatant is taken.
(2) serum storage: for short-term storage, the supernatant can be transferred to EP tube and stored in the refrigerator at -80°C; for long-term storage, the supernatant can be stored in a cryotube in liquid nitrogen.
(3) detection (refer to the Biovision EZQuant dsDNA operating instruction) preparation: after the serum sample has been collected, the detection would be carried out. 1x TE buffer, 2x dsDNA staining solution and 10 ng/µl of standard are prepared according to the instruction in the kit, respectively.
(4) standard curve preparation: the linear standard is prepared by formulating 10 ng/µl of standard according to the Biovision EZQuant dsDNA instruction. 0, 2, 4, 6, 8 and 10 µl of standard sample is added to corresponding well of the black microtiter plate, respectively. The standard sample is made up to 50 µl with 1x TE buffer. Three replicates are set for each concentration.
(5) sample preparation: 10 µl of the sample is added to each well of the black microtiter plate and then 1x TE buffer is used to make up to 50 µl. Three replicates are set for each concentration.
(6) staining: 50 µl of 2x dsDNA staining solution is added to each well, the black microtiter plate is placed on a shaker and gently shaken at room temperature for 5 minutes away from light.
(7) detection: after the staining reaction is completed, the microplate reader with Ex/Em = 480/530 detection module or similar apparatus is used for immediate detection.
(8) calibration: each plate with wells should be designed with a standard curve and a 0 hole. After the detection results come out, the standard curve and the 0 hole is firstly used for calibration. Generally, if the r2 value of the linear correlation relationship of the standard curve should be greater than 0.99, the detection result is good, and the data can be used.
(9) statistical analysis of data: SPSS 23.0 and R Version 4.0.2 software are used for data processing and analysis. All tests use two-sided tests, *P* value less than 0.05 is considered as statistically significant.

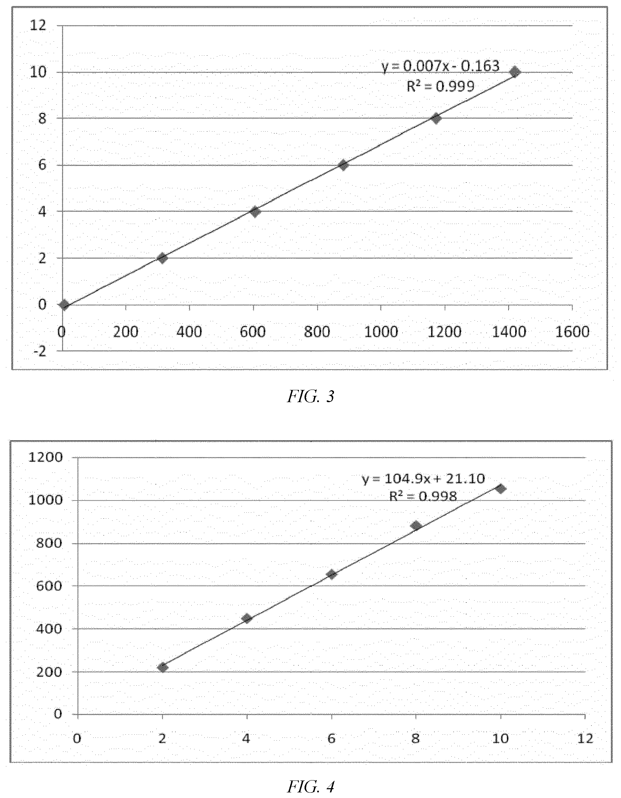

In examples of the present invention, quality control on the stability of the fluorescence quantitative detection on serum dsDNA is performed. Two types of detection apparatus, biotek cell vision5 and flex station3, are used to compare and analyse the standard curve. r2 is 0.999 and 0.998, respectively, indicating that the standard curve is well prepared, and there is no variability in the direct detection results of the instruments (as shown in Figure 3 and Figure 4).

### Example 1 Serum dsDNA distribution tendency of psoriasis patients and normal people with different characteristics

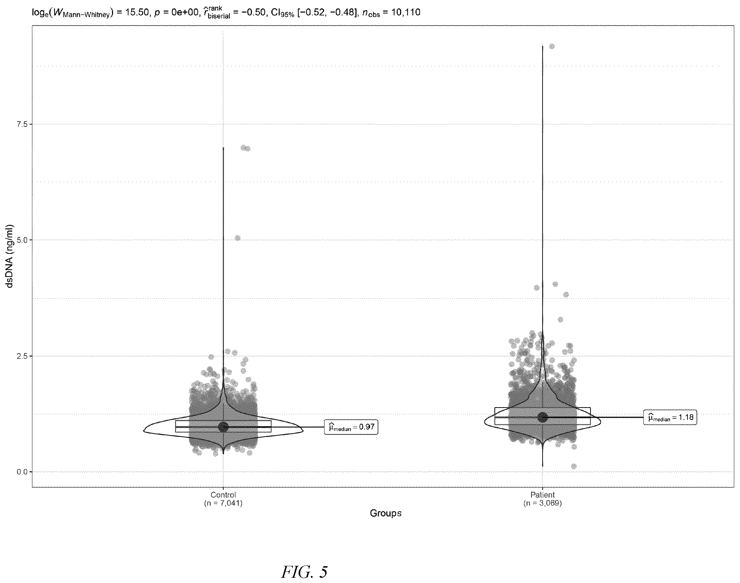

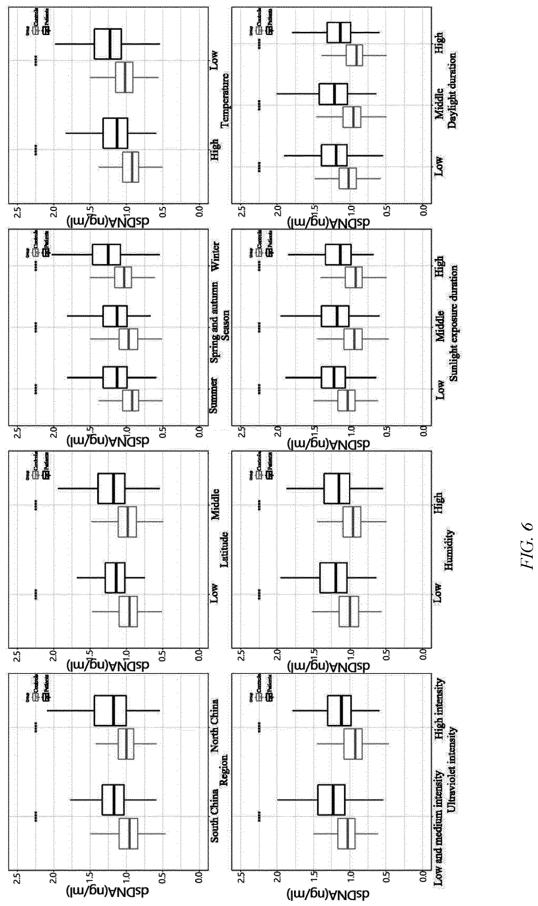

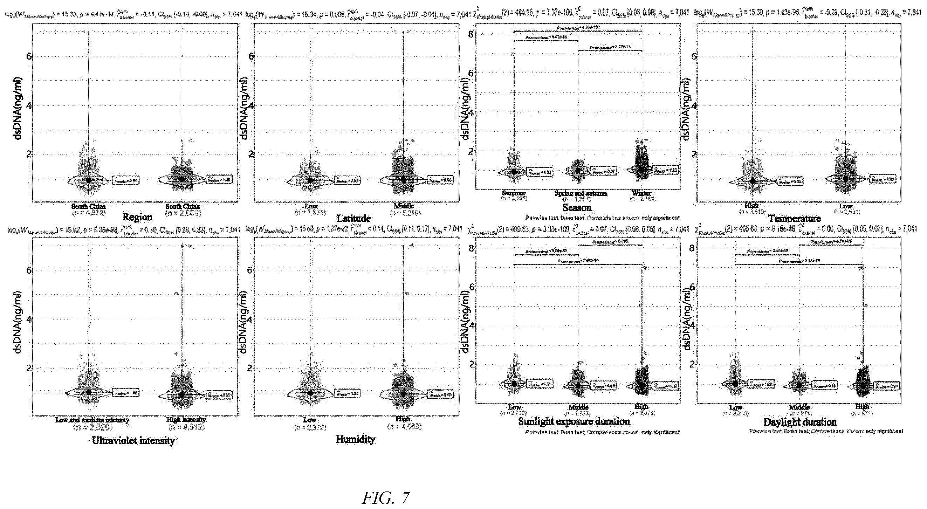

(1) The level of serum dsDNA in the psoriasis group was significantly higher than that in the control group (P< 0.001) (Figure 5). When stratified by region, latitude, season, temperature, ultraviolet light, humidity, sunlight exposure duration, and sunlight exposure duration, the differences in serum dsDNA levels between the psoriasis group and the control group were also significant (P< 0.001) (Figure 6).
(2) The median level of serum dsDNA in the control group was 0.97 ng/ml (interquartile range: 0.86-1.11 ng/ml). Healthy controls who were male, over 40 years old, in winter, in northern region, in mid-latitude region, in low temperature region, in low ultraviolet region, in low humidity region, in short sunlight exposure duration region, and in short daylight duration region had significantly higher serum dsDNA levels. (Figure 7, Table 1-Table 3)

**Table 1 Comparison of serum dsDNA of participants (all participants (n = 10,110)) with different characteristics**

| | All participants (n = 10,110) | | |
|---|---|---|---|
| | dsDNA (median, IQR) | Z/_{X}2 | P |
| Age (years) | | | |
| ≤ 40 | 1.02 (0.88, 1.18) | -3.323 | 0.001 |
| > 40 | 1.03 (0.90, 1.19) | | |
| Gender | | | |
| Male | 1.05 (0.91, 1.22) | -10.771 | < 0.001 |
| Female | 1.00 (0.87, 1.15) | | |
| Region | | | |
| South China | 1.01 (0.87, 1.17) | -12.621 | < 0.001 |
| North China | 1.05 (0.93, 1.22) | | |
| Temperature | | | |
| High | 0.97 (0.86, 1.14) | -20.226 | < 0.001 |
| Low | 1.07 (0.94, 1.23) | | |
| Season | | | |
| Summer | 0.96 (0.85, 1.13) | 616.188 | < 0.001 |
| Spring and autumn | 1.02 (0.88, 1.16) | | |
| Winter | 1.09 (0.96, 1.26) | | |
| Latitude | | | |
| Low | 1.00 (0.87, 1.14) | -7.588 | < 0.001 |
| Middle | 1.03 (0.90, 1.20) | | |
| Ultraviolet intensity | | | |
| Medium and low | 1.09 (0.97, 1.27) | -27.236 | < 0.001 |
| High | 0.97 (0.85, 1.13) | | |
| Humidity | | | |
| Low | 1.20 (1.04, 1.43) | -16.789 | < 0.001 |
| High | 1.00 (0.87, 1.14) | | |
| Daylight duration | | | |
| Short | 1.07 (0.95, 1.23) | 367.883 | < 0.001 |
| Medium | 1.03 (0.87, 1.19) | | |
| Long | 0.97 (0.85, 1.13) | | |
| Sunlight exposure duration | | | |
| Short | 1.04 (0.93, 1.18) | 421.906 | < 0.001 |
| Medium | 1.09 (0.92, 1.30) | | |
| Long | 0.97 (0.85, 1.13) | | |
| Abbreviation: dsDNA, double-stranded DNA. | | | |

**Table 2 Comparison of serum dsDNA of participants (patients (n = 3069)) with different characteristics**

| | Patients (n = 3069) | | |
|---|---|---|---|
| | dsDNA (median, IQR) | *Z*/*_{X}²* | *P* |
| Age (years) | | | |
| ≤ 40 | 1.17 (1.02, 1.39) | -0.044 | 0.965 |
| > 40 | 1.18 (1.02, 1.39) | | |
| Gender | | | |
| Male | 1.19 (1.03, 1.39) | -1.681 | 0.093 |
| Female | 1.16 (1.00, 1.39) | | |
| Region | | | |
| South China | 1.17 (1.04, 1.34) | -1.789 | 0.074 |
| North China | 1.19 (1.00, 1.45) | | |
| Temperature | | | |
| High | 1.13 (0.99, 1.33) | -9.884 | < 0.001 |
| Low | 1.23 (1.07, 1.45) | | |
| Season | | | |
| Summer | 1.14 (0.99, 1.33) | 123.039 | < 0.001 |
| Spring and autumn | 1.13 (0.99, 1.33) | | |
| Winter | 1.25 (1.08, 1.48) | | |
| Latitude | | | |
| Low | 1.14 (1.01, 1.29) | -3.597 | < 0.001 |
| Middle | 1.19 (1.02, 1.41) | | |
| Ultraviolet intensity | | | |
| Medium and low | 1.24 (1.07, 1.46) | -11.239 | < 0.001 |
| High | 1.12 (0.98, 1.31) | | |
| Humidity | | | |
| Low | 1.20 (1.04, 1.43) | -5.189 | < 0.001 |
| High | 1.15 (1.00, 1.36) | | |
| Daylight duration | | | |
| Short | 1.22 (1.06, 1.40) | 28.234 | < 0.001 |
| Medium | 1.18 (1.02, 1.41) | | |
| Long | 1.14 (0.99, 1.35) | | |
| Sunlight exposure duration | | | |
| Short | 1.19 (1.04, 1.39) | 34.962 | < 0.001 |
| Medium | 1.22 (1.04, 1.43) | | |
| Long | 1.14 (1.00, 1.33) | | |
| Abbreviation: dsDNA, double-stranded DNA. | | | |

**Table 3 Comparison of serum dsDNA of participants with different characteristics (comparative example (n = 7041))**

| | Comparative example (n = 7041) | | |
|---|---|---|---|
| | dsDNA (median, IQR) | *Z*/*_{X}²* | *P* |
| Age (years) | | | |
| ≤ 40 | 0.96 (0.84, 1.10) | -4.846 | < 0.001 |
| > 40 | 0.98 (0.87, 1.11) | | |
| Gender | | | |
| Male | 0.98 (0.87, 1.12) | 6.009 | < 0.001 |
| Female | 0.96 (0.85, 1.09) | | |
| Region | | | |
| South China | 0.96 (0.84, 1.10) | -7.531 | < 0.001 |
| North China | 1.00 (0.89, 1.11) | | |
| Temperature | | | |
| High | 0.92 (0.83, 1.05) | -20.866 | < 0.001 |
| Low | 1.02(0.91,1.15) | | |
| Season | | | |
| Summer | 0.92 (0.83, 1.05) | 484.431 | < 0.001 |
| Spring and autumn | 0.97 (0.84. 1.10) | | |
| Winter | 1.03 (0.93, 1.16) | | |
| Latitude | | | |
| Low | 0.96 (0.85, 1.10) | -2.667 | 0.008 |
| Middle | 0.98 (0.86, 1.11) | | |
| Ultraviolet intensity | | | |
| Medium and low | 1.03 (0.93, 1.16) | -21.013 | < 0.001 |
| High | 0.93 (0.83, 1.07) | | |
| Humidity | | | |
| Low | 1.00 (0.88, 1.15) | -9.780 | < 0.001 |
| High | 0.96 (0.84, 1.09) | | |
| Daylight duration | | | |
| Short | 1.03 (0.93, 1.16) | 499.733 | < 0.001 |
| Medium | 0.94 (0.83, 1.08) | | |
| Long | 0.92 (0.83, 1.06) | | |
| Sunlight exposure duration | | | |
| Short | 1.02 (0.91, 1.14) | 405.716 | < 0.001 |
| Medium | 0.94 (0.85, 1.09) | | |
| Long | 0.91 (0.82, 1.05) | | |
| Abbreviation: dsDNA, double-stranded DNA. | | | |

(3) The median level of serum dsDNA in psoriasis group was 1.18 ng/ml (interquartile range: 1.02-1.39 ng/ml). Psoriasis patients who were in winter, in northern region, in mid-latitude region, in low temperature region, in low ultraviolet region, in low humidity region, in short sunlight exposure duration region, and in short daylight duration region had significantly elevated serum dsDNA levels (Table 1).
(4) Among psoriasis patients, the serum dsDNA levels of patients with smoking and alcohol use habits were significantly higher than those of patients without these characteristics. Psoriasis patients with different types of psoriasis had different serum dsDNA levels (Table 4).

**Table 4 Comparison of serum dsDNA of patients with different characteristics**

| Characteristics | dsDNA (ng/ml, median [IQR]) | *Z*/*_{X}²* | P |
|---|---|---|---|
| Life habits | | | |
| Smoking | | | |
| Yes (n = 952) | 1.21 (1.05, 1.43) | -4.666 | < 0.001 |
| No (n = 2117) | 1.16 (1.00, 1.37) | | |
| Alcohol use | | | |
| Yes (n = 775) | 1.20 (1.05, 1.40) | -2.957 | 0.003 |
| No (n = 2294) | 1.17 (1.01, 1.38) | | |
| Medication history | | | |
| Systemic medication | | | |
| Yes (n = 1815) | 1.17(1.00, 1.40) | -1.906 | 0.057 |
| No (n = 1254) | 1.19 (1.04, 1.38) | | |
| External medication | | | |
| Yes (n = 2726) | 1.18 (1.02, 1.39) | -1.158 | 0.247 |
| No (n = 343) | 1.18(1.03, 1.42) | | |
| Disease characteristics | | | |
| Types of psoriasis | | | |
| Normal type (n = 1566) | 1.12 (0.99, 1.31) | 9.459 | 0.024 |
| Arthropathica (n = 43) | 1.16 (0.94, 1.47) | | |
| Pustule (n = 31) | 1.15 (0.97, 1.34) | | |
| Erythrodermic (n = 37) | 1.24 (1.10, 1.42) | | |
| Course of psoriasis | | | |
| Active stage (n = 1496) | 1.25(1.10, 1.47) | 24.490 | < 0.001 |
| Resting stage (n = 584) | 1.18 (1.04, 1.37) | | |
| Regression stage (n = 93) | 1.16 (1.02, 1.37) | | |
| Abbreviation: dsDNA, double-stranded DNA | | | |

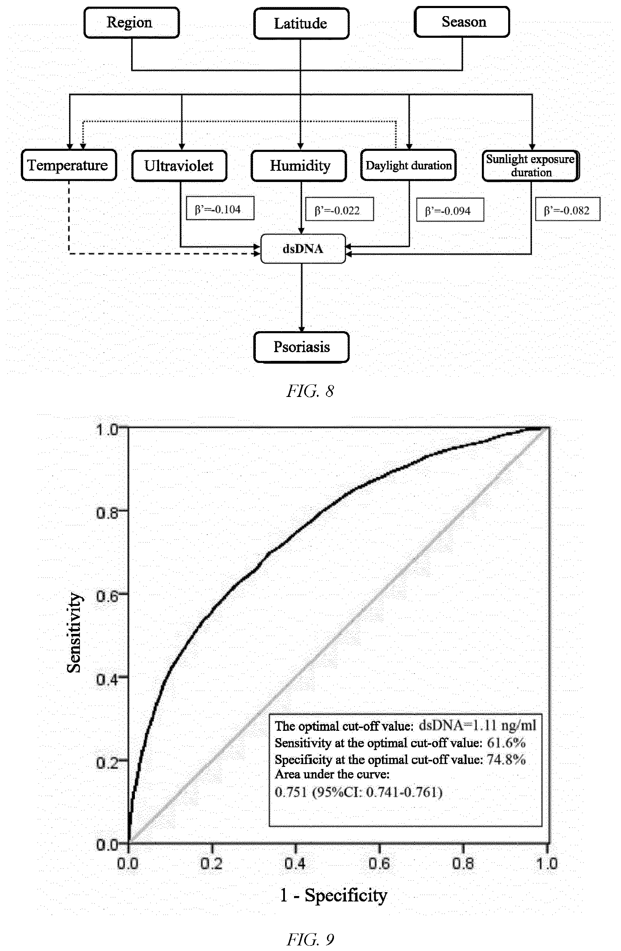

(5) The participants' serum dsDNA levels were subjected to natural logarithmic transformation to explore the contribution of climate factors. The linear regression results show that ultraviolet light, humidity, sunlight exposure duration and daylight duration are negatively correlated with serum dsDNA levels, and ultraviolet light is the biggest contributor (Figure 8).

### Example 2 The clinical predictive value of serum dsDNA in psoriasis

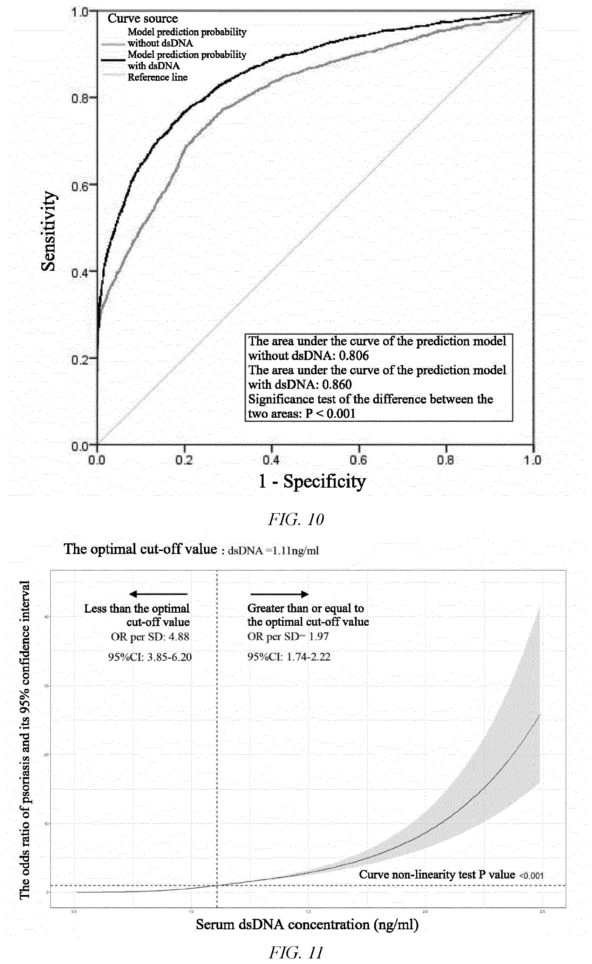

(1) Among all participants, according to the ROC curve, the optimal cut-off value for serum dsDNA to predict psoriasis was 1.11 ng/ml, the cut-off value predicted psoriasis with a sensitivity and specificity of 61.6% and 74.8%, respectively (Figure 9).
(2) Logistic regression model was further constructed to predict psoriasis (independent variables comprised age, gender, BMI, region, latitude, season, temperature, ultraviolet light, humidity, sunlight exposure duration, daylight duration). After adding dsDNA to the multivariate logistic regression model, the prediction effect increased, and the area under the curve (AUC) increased from 0.806 to 0.860 (Figure 10). According to the optimal cut-off value for serum dsDNA, serum dsDNA has a good prediction for psoriasis in participants with different characteristics (Table 5).

**Table 5 The sensitivity and specificity of dsDNA cut-off value (1.11 ng/ml) to identify the psoriasis of all participants with different characteristics**

| Group | | Psoriasis | Sensitivity | Specificity |
|---|---|---|---|---|
| | | | Ratio (95% CI) | Ratio (95% CI) |
| All participants | | 3069 | 61.6% (59.9%-634%) | 74.8% (73.8%-75.8%) |
| Age of patients | | | | |
| | ≤ 40 years | 1429 | 61.3% (58.7%-63.8%) | 76.3% (74.8%-77.8%) |
| | > 40 years | 1640 | 61.9% (59.5%-64.3%) | 73.5% (72.1%-74.9%) |
| Gender of patients | | | | |
| | Male | 2008 | 62.8% (60.7%-65.0%) | 72.6% (71.1%-74.0%) |
| | Female | 1061 | 59.4% (56.3%-62.3%) | 77.2% (75.7%-78.6%) |
| Temperature | | | | |
| | High | 1572 | 54.4% (51.9%,-5679%) | 81.6% (80.3%-82.5%) |
| | Low | 1497 | 69.3% (66.8%-71.6%) | 68.1% (66.5%-69,6%) |
| Season | | | | |
| | Summer | 1067 | 54.8% (51.8%-57.8%) | 81.1% (79.7%-82.5%) |
| | Spring and autumn | 744 | 54.7% (51.0%-58.3%) | 76.6% (74.2%-78.8%) |
| | Winter | 1258 | 71.5% (68.9%-74.0%) | 65.8% (63.9%-67.6%) |
| Region | | | | |
| | South China | 1524 | 63.0% (60.5%-65.4%) | 75.2% (73.9%-76.3%) |
| | North China | 1545 | 60.3% (57.8%-62.8%) | 74.0% (72.0%-75.9%) |
| Latitude | | | | |
| | Low | 489 | 56.6% (52.1%-61.1%) | 76.2% (74.2%-78.2%) |
| | Middle | 2580 | 62.6% (60.7%-64.5%) | 74.3% (73.1%-75.5%) |

**Table 6 NPV, PPV and consistency ratio of dsDNA cut-off value to identify the psoriasis of all participants with different characteristics**

| Group | PPV | NPV | Consistency ratio | | |
|---|---|---|---|---|---|
| | Ratio (95% CI) | Ratio (95% CI) | | | |
| All participants | 51.6% (50.0%-53.2%) | 81.7% (80.8%-82.7%) | 70.8% | | |
| Age of participants | | | | | |
| ≤ 40 years | 53.3% (50.9%-55.8%) | 81.7% (80.2%-83.0%) | 71.7% | | |
| > 40 years | 50.2% (48.0%-52.4%) | 81.8% (80.4%-83.0%) | 70.0% | | |
| Gender of participants | | | | | |
| Male | 55.9% (53.8%-58.0%) | 77.9% (76.5%-79.3%) | 69.1% | | |
| Female | 44.7% (42.1%-47.4%) | 85.9% (84.6%-87.1%) | 72.9% | | |
| Temperature | | | | | |
| High | 57.0% (54.4%-59.5%) | 80.0% (78.6%-81.3%) | 73.2% | | |
| Low | 47.9% (45.6%-50.0%) | 83.9% (82.5%-85.2%) | 68.4% | | |
| Season | | | | | |
| Summer | 49.2% (46.4%-52.1 %) | 84.3% (83.0%-85.6%) | 74.5% | | |
| Spring and autumn | 56.1% (52.4%-59.8%) | 75.5% (73.1%-77.7%) | 68.8% | | |
| Winter | 51.4% (49.0%-53.7%) | 48.6% (46.3%-51.0%) | 67.7% | | |
| Region | | | | | |
| South China | 43.7% (41.6%-45,8%) | 86.9% (85.8%-87.9%) | 72.3% | | |
| North China | 63.4% (60.9%-65.8%) | 71.4%(69.4%-73.3%) | 68.2% | | |
| Latitude | | | | | |
| Low | 38.9% (28.8%-32.6%) | 86.8% (85.0%-88.4%) | 70.8% | | |
| Middle | 54.7% (52.9%-56.5%) | 80.0%(78.9%-81.2%) | 70.4% | | |
| Abbreviations: dsDNA, double-stranded DNA; NPV: negative predictive value; PPV, positive predictive value. | | | | | |

### Example 3 Serum dsDNA increasing the risk of occurrence of psoriasis

(1) After adjusting age, gender, BMI, region, latitude, temperature, season, ultraviolet light, humidity, sunlight exposure duration and daylight duration, the Logistic regression model showed that for every 1 ng/ml increase in serum dsDNA, the psoriasis risk increased by 40.40 times (OR: 41.40, 95% CI: 32.32-53.03). After serum dsDNA quartile segmentation, it was found that the incidence of psoriasis increased with the increase of serum dsDNA quartile level, and there was a significant dose-response relationship. Similar results, *i.e.,* positive correlation between serum dsDNA level and the risk of occurrence of psoriasis, were found in the subgroups of region, latitude, temperature, season, ultraviolet intensity, humidity, sunlight exposure duration, and sunlight exposure duration (Table 7).

**Table 7 The relationship between dsDNA and the risk of occurrence of psoriasis**

| → | | | | | |
|---|---|---|---|---|---|
| Subgroups | dsDNA (ng/ml, median [interval]) | Case | Comparative example | | |
| All categories | Q1 (0.81, [< 0.89]) | 258 | 2209 | | |
| | Q2 (0.96, [0.89-1.02]) | 531 | 2042 | | |
| | Q3 (1.10, [1.03-1.18]) | 778 | 1755 | | |
| | Q4 (1.35, [> 1.18]) | 1502 | 1035 | | |
| Latitude | Low | | | | |
| | Q1 (0.80, [< 0.87]) | 18 | 549 | | |
| | Q2 (0.93, [0.87-0.99]) | 79 | 474 | | |
| | Q3 (1.06, [1.00-1.14]) | 152 | 482 | | |
| | Q4 (1.30, [> 1.14]) | 240 | 326 | | |
| | Middle | | | | |
| | Q1 (0.82, [< 0.90]) | 255 | 1682 | | |
| | Q2 (0.96, [0.90-1.02]) | 397 | 1450 | | |
| | Q3 (1.10, [1.03-1.20]) | 696 | 1374 | | |
| | Q4 (1.37, [> 1.20]) | 1232 | 704 | | |
| Temperature | High | | | | |
| | Q1 (0.79, [< 0.86]) | 120 | 1146 | | |
| | Q2 (0.91, [0.86-0.96]) | 223 | 979 | | |
| | Q3 (1.05, [0.97-1.14]) | 481 | 903 | | |
| | Q4 (1.30, [> 1.14]) | 748 | 482 | | |
| | Low | | | | |
| | Q1 (0.77, [< 0.94]) | 126 | 1045 | | |
| | Q2 (1.00, [0.94-1.06]) | 242 | 1070 | | |
| | Q3 (1.14, [1.07-1.23]) | 387 | 952 | | |
| | Q4 (1.40, [> 1.23]) | 742 | 464 | | |
| Ultraviolet intensity | Medium and low | | | | |
| | Q1 (0.86, [< 0.97]) | 171 | 841 | | |
| | Q2 (1.02, [0.97-1.08]) | 264 | 721 | | |
| | Q3 (1.16, [1.09-1.27]) | 444 | 653 | | |
| | Q4 (1.44, [> 1.27]) | 711 | 314 | | |
| | High | | | | |
| | Q1 (0.78, [< 0.85]) | 95 | 1280 | | |
| | Q2 (0.90, [0.85-0.96]) | 235 | 1275 | | |
| | Q3 (1.04, [0.97-1.13]) | 432 | 1233 | | |
| | Q4 (1.275 [> 1.13]) | 717 | 724 | | |
| Season | Summer | | | | |
| | Q1 (0.78, [< 0.85]) | 63 | 943 | | |
| | Q2 (0.89, [0.85-0.95]) | 140 | 927 | | |
| | Q3 (1.04, [0.96-1.13]) | 325 | 837 | | |
| | Q4 (1.28, [> 1.13]) | 539 | 488 | | |
| | Spring and autumn | | | | |
| | Q1 (0.80, [< 0.88]) | 65 | 445 | | |
| | Q2 (0.95, [0.88-1.01]) | 166 | 334 | | |
| | Q3 (1.09, [1.02-1.16]) | 187 | 398 | | |
| | Q4 (1.295 [> 1.16]) | 326 | 180 | | |
| | Winter | | | | |
| | Q1 (0.88, [< 0.96]) | 114 | 768 | | |
| | Q2 (1.02, [0.96-1.08]) | 213 | 762 | | |
| | Q3 (1.16, [1.09-1.26]) | 328 | 628 | | |
| | Q4 (1.45, [> 1.26]) | 603 | 331 | | |
| Daylight duration | Short | | | | |
| | Q1 (0.87, [< 0.95]) | 99 | 786 | | |
| | Q2 (1.01, [0.95-1.06]) | 158 | 803 | | |
| | Q3 (1.14, [1.07-1.23]) | 266 | 716 | | |
| | Q4 (1.38, [> 1.23]) | 470 | 425 | | |
| | Medium | | | | |
| | Q1 (0.79, [< 0.87]) | 76 | 635 | | |
| | Q2 (0.95, [0.87-1.02]) | 245 | 549 | | |
| | Q3 (1.10, [1.03-1.19]) | 318 | 464 | | |
| | Q4 (1.38, [> 1.19]) | 574 | 185 | | |
| | Long | | | | |
| | Q1 (0.78, [< 0.85]) | 50 | 733 | | |
| | Q2 (0.90, [0.85-0.96]) | 139 | 739 | | |
| | Q3 (1.04, [0.97-1.13]) | 236 | 650 | | |
| | Q4 (1.29, [> 1.13]) | 438 | 356 | | |
| Sunlight exposure duration | Short | | | | |
| | Q1 (0.84, [< 0.93]) | 71 | 939 | | |
| | Q2 (0.98, [0.93-1.03]) | 89 | 862 | | |
| | Q3 (1.10, [1.04-1.18]) | 171 | 951 | | |
| | Q4 (1.32, [> 1.18]) | 332 | 637 | | |
| | Medium | | | | |
| | Q1 (0.85, [< 0.92]) | 139 | 425 | | |
| | Q2 (1.00, [0.92-1.08]) | 309 | 291 | | |
| | Q3 (1.18, [1.09-1.30]) | 426 | 201 | | |
| | Q4 (1.48, [> 1.30]) | 537 | 54 | | |
| | Long | | | | |
| | Q1 (0.77, [< 0.85]) | 64 | 834 | | |
| | Q2 (0.90, [0.85-0.96]) | 136 | 796 | | |
| | Q3 (1.04, [0.97-1.13]) | 295 | 680 | | |
| | Q4 (1.28, [> 1.13]) | 500 | 371 | | |
| Region | South China | | | | |
| | Q1 (0.79, [< 0.87]) | 87 | 1509 | | |
| | Q2 (0.94, [0.87-1.00]) | 212 | 1417 | | |
| | Q3 (1.08, [1.01-1.17]) | 469 | 1238 | | |
| | Q4 (1.32, [> 1.17]) | 756 | 808 | | |
| | North China | | | | |
| | Q1 (0.86, [< 0.93]) | 110 | 369 | | |
| | Q2 (0.99, [0.93-1.04]) | 284 | 678 | | |
| | Q3 (1.12, [1.05-1.22]) | 359 | 720 | | |
| | Q4 (1.43, [> 1.22]) | 792 | 302 | | |
| Humidity | Low | | | | |
| | Q1 (0.85, [< 0.93]) | 170 | 815 | | |
| | Q2 (1.00, [0.93-1.06]) | 295 | 674 | | |
| | Q3 (1.15, [1.07-1.26]) | 464 | 572 | | |
| | Q4 (1.46, [> 1.26]) | 666 | 311 | | |
| | High | | | | |
| | Q1 (0.79, [< 0.87]) | 107 | 1381 | | |
| | Q2 (0.93, [0.87-0.99]) | 234 | 1285 | | |
| | Q3 (1.06, [1.00-1.14]) | 383 | 1228 | | |
| | Q4 (1.28, [> 1.14]) | 750 | 775 | | |
| → | | | | | |

| Model 1 t | | Model 2‡ | | Model 3§ | |
|---|---|---|---|---|---|
| OR (95% CI) | *P* tende ncy | OR (95% CI) | *P* tende ncy | OR (95% CI) | *P* tenden cy |
| Parameters | | Parameters | | Parameters | |
| 2.23 (1.90, 2.61) | | 2.23 (1.89, 2.62) | | 2.41 (2.00, 2.89) | |
| 3.80 (3.25, 4.43) | | 3.80 (3.25, 4.44) | | 4.79 (3.99, 5.74) | |
| 12.42 (10.68, 14.45) | < 0.001 | 12.61 (10.81, 14.70) | < 0.001 | 14.61 (12.18, 17.53) | < 0.001 |
| | | | | | |
| Parameters | | Parameters | | Parameters | |
| 5.08 (3.00, 8.61) | | 4.97 (2.92, 8.46) | | 7.09(3.30, 15.25) | |
| 9.62 (5.81, 15.92) | | 9.47 (5.69, 15.77) | | 13.21 (6.05, 28.87) | |
| 22.45 (13.64, 36.95) | < 0.001 | 21.73 (13.10, 36.02) | < 0.001 | 26.39 (12.07, 57.71) | < 0.001 |
| | | | | | |
| Parameters | | Parameters | | Parameters | |
| 1.81 (1.52, 2.14) | | 1.80 (1.51, 2.14) | | 1.87 (1.50, 2.34) | |
| 3.34 (2.85, 3.92) | | 3.35 (2.85, 3.94) | | 3.64 (2.95, 4.49 | |
| 11.54 (9.83, 13.56) | < 0.001 | 11.78 (10.00, 13.86) | < 0.001 | 13.16 (10.60, 16.34) | < 0.001 |
| | | | | | |
| Parameters | | Parameters | | Parameters | |
| 2.17 (1.71, 2.76) | | 2.26 (1.78, 2.88) | | 2.15 (1.61, 2.86) | |
| 5.09 (4.09, 6.33) | | 5.25 (4.21, 6.55) | | 6.56 (4.98, 8.63) | |
| 14.82 (11.89, 18.47) | < 0.001 | 15.52 (12.40, 19.43) | < 0.001 | 23.57 (17.68, 31.43) | < 0.001 |
| | | | | | |
| Parameters | | Parameters | | Parameters | |
| 1.88 (1.49, 2.36) | | 1.85 ( 1.47, 2.34) | | 1.78 (1.34,2.36) | |
| 3.37 (2.71, 4.20) | | 3.25 (2.61,4.06) | | 2.60 (1.98, 3.40) | |
| 13.26 (10.66, 16.50) | < 0.001 | 13.27 (10.64, 16.55) | < 0.001 | 9.18 (7.00, 12.05) | < 0.001 |
| | | | | | |
| Parameters | | Parameters | | Parameters | |
| 1.80 (1.45, 2.24) | | 1.80 (1.45, 2.24) | | 1.64 (1.20, 2.24) | |
| 3.34 (2.73, 4.10) | | 3.28 (2.67, 4.04) | | 2.63 (1.96, 3.53) | |
| 11.14 (9.01, 13.76) | < 0.001 | 11.70 (9.43, 14.50) | < 0.001 | 9.82 (7.31, 13.18) | < 0.001 |
| | | | | | |
| Parameters | | Parameters | | Parameters | |
| 2.48 (1.93, 3.19) | | 2.54 (1.97, 3.26) | | 2.36 (1.76,3.15) | |
| 4.72 (3.73, 5.97) | | 4.81 (3.80, 6.10) | | 7.00 (5.27, 9.29) | |
| 13.34 (10.57, 16.84) | < 0.001 | 13.32 (10.53, 16.86) | < 0.001 | 21.39 (16.04, 28.54) | < 0.001 |
| | | | | | |
| Parameters | | Parameters | | Parameters | |
| 2.26 (1.66,,3.108) | | 2.45 (1.79, 3,35) | | 1.84 (1.31, 2.59) | |
| 5.81 (4.37, 7.73) | | 6.20 (4.64, 8.27) | | 5.39 (3.90, 7.44) | |
| 16.53 (12.46, 21.94) | < 0.001 | 17.90 (13.42, 23.88) | < 0.001 | 17.69 (12.76, 24.52) | < 0.001 |
| | | | | | |
| Parameters | | Parameters | | Parameters | |
| 3.40 (2.47, 4.69) | | 3.22 (2.33, 4.47) | | 6.80 (2.67, 17.35) | |
| 3.22 (2.35, 4.40) | | 3.12 (2.26, 4.29) | | 10.19 (4.05, 25.65) | |
| 12.40 (9.02, 17.03) | < 0.001 | 12.07 (8.70, 16.73) | < 0.001 | 17.53 (7.04, 43.70) | < 0.001 |
| | | | | | |
| Parameters | | Parameters | | Parameters | |
| 1.88 (1.47, 2.41) | | 1.89 (1.47, 2.43) | | 1.49 (1.08, 2.05) | |
| 3.52 (2.77, 4.46) | | 3.46 (2.72, 4.40) | | 2.30 (1.69, 3.13) | |
| 12.27 (9.67, 15.57) | < 0.001 | 12.79 (10.04, 16.28) | < 0.001 | 8.61 (6.36, 11.65) | < 0.001 |
| | | | | | |
| Parameters | | Parameters | | Parameters | |
| 1.56 (1.19, 2.05) | | 1.56 (1.19, 2.05) | | 1.54 (1.12, 2.11) | |
| 2.95 (2.29, 3.79) | | 2.85 (1.21, 3.68) | | 2.28 (1.68, 3.07) | |
| 8.78 (6.86, 11.24) | < 0.001 | 8.91 (6.94, 11.45) | < 0.001 | 8.10 (6.06, 10.81) | < 0.001 |
| | | | | | |
| Parameters | | Parameters | | Parameters | |
| 3.73 (2.81, 4.94) | | 3.74 (2.81, 4.97) | | 5.77 (3.42, 9.73) | |
| 5.73 (4.34, 7.56) | | 5.64 (4.26, 7.47) | | 11.08 (6.45, 19.02) | |
| 25.92 (19.40, 34.64) | < 0.001 | 27.40 (20.35, 36.90) | < 0.001 | 42.00 (23.82, 74.03) | < 0.001 |
| | | | | | |
| Parameters | | Parameters | | Parameters | |
| 2.76(1.96, 3.87) | | 2.83 (2.01, 3.97) | | 1.96 (1.34, 2.87) | |
| 5.32 (3.85, 7.35) | | 5.46 (3.95, 7.55) | | 4.11 (2.85, 5.91) | |
| 18.04 (13.11, 24.81) | < 0.001 | 18.29 (13.25, 25.24) | < 0.001 | 11.07 (7.64, 16.06) | < 0.001 |
| | | | | | |
| Parameters | | Parameters | | Parameters | |
| 1.37 (0.99, 1.89) | | 1.37 (0.99, 1.90) | | 1.25 (0.83, 1.91 | |
| 2.38 (1.78, 3.18) | | 2.29 (1.70, 3.06) | | 1.85 (1.26, 2.71) | |
| 6.89 (5.23, 9.08) | < 0.001 | 6.71 (5.08, 8.86) | < 0.001 | 6.60 (4.66, 9.34) | < 0.001 |
| | | | | | |
| Parameters | | Parameters | | Parameters | |
| 3.25 (2.53, 4.17) | | 3.39 (2.62, 4.40) | | 4.28 (2.79, 6.59) | |
| 6.48 (5.02, 8.36) | | 7.69 (5.87, 10.06) | | 9.58 (6.10, 15.03) | |
| 30.41 (21.66, 42.68) | < 0.001 | 40.92 (28.54, 58.66) | < 0.001 | 34.79 (20.45, 59.17) | < 0.001 |
| | | | | | |
| Parameters | | Parameters | | Parameters | |
| 2.23 (1.63, 3.04) | | 2.30 (1.68, 3.15) | | 2.02 (1.42, 2.89) | |
| 5.65 (4.24, 7.54) | | 5.79 (4.33, 7.74) | | 4.64 (3.31, 6.49) | |
| 17.56 (13.17, 23.41) | < 0.001 | 17.91 (13.40, 23.94) | < 0.001 | 13.60 (9.67, 19.12) | < 0.001 |
| | | | | | |
| Parameters | | Parameters | | Parameters | |
| 2.59 (2.00, 3.36) | | 2.56 (1.97, 3.32) | | 2.66 (1.95, 3.62) | |
| 6.57 (5.16, 8.36) | | 6.50 (5.11, 8.29) | | 7.40 (5.48, 9.99) | |
| 16.23 (12.79, 20.58) | < 0.001 | 16.08 (12.66, 20.44) | < 0.001 | 17.77 (13.17, 23.96) | < 0.001 |
| | | | | | |
| Parameters | | Parameters | | Parameters | |
| 1.40 (1.14, 1.73) | | 1.38 (1.11, 1.71) | | 2.00 (1.51, 2.65) | |
| 1.67 (1.36, 2.04) | | 1.67 (1.36, 2.06) | | 2.47 (1.87, 3.27) | |
| 12.28 (9.81, 15.37) | < 0.001 | 12.96 (10.28, 16.35) | < 0.001 | 18.38 (13.37, 25.26) | < 0.001 |
| | | | | | |
| Parameters | | Parameters | | Parameters | |
| 2.09 (1.68, 2.59) | | 2.10 (1.69, 2.61) | | 1.74 (1.31, 2.32) | |
| 3.88 (3.16, 4.77) | | 3.95 (3.21, 4.87) | | 2.89 (2.18, 3.83) | |
| 10.20 (8.24, 12.63) | < 0.001 | 10.73 (8.63, 13.33) | < 0.001 | 6.99 (5.22, 9.36) | < 0.001 |
| | | | | | |
| Parameters | | Parameters | | Parameters | |
| 2.35 (1.85, 2.99) | | 2.33 (1.83, 2.97) | | 2.40 (1.82, 3.16) | |
| 4.02 (3.21, 5.05) | | 4.01 (3.19, 5.05) | | 6.72 (5.09, 8.88) | |
| 12.49 (10.01, 15.58) | < 0.001 | 12.17 (9.74, 15.21) | < 0.001 | 21.97 (16.71, 28.89) | < 0.001 |
| † Model 1 had no variables adjusted | | | | | |
| ‡ Model 2 had adjusted variables except for age, gender, season, region, and body mass index (variables in this subgroup were not adjusted). | | | | | |
| § Model 3 had adjusted variables except for age, gender, season, region, body mass index, temperature, ultraviolet degree, humidity, latitude, sunlight exposure duration, daylight duration (variables in this subgroup were not adjusted). | | | | | |

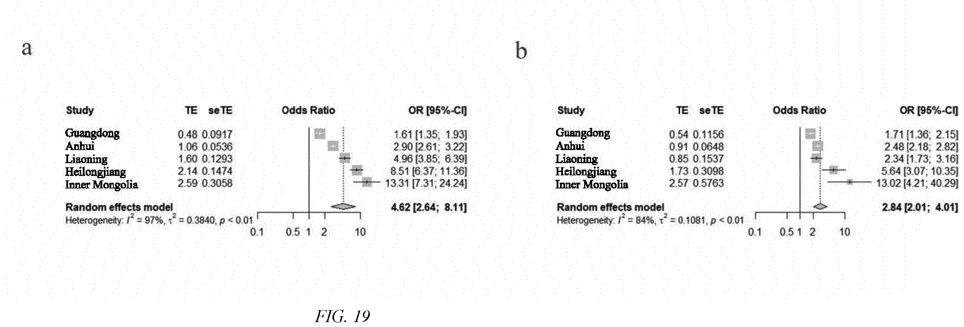

(2) With the increase of serum dsDNA, the OR of psoriasis gradually increased (Figure 11). When serum dsDNA < 1.11 ng/ml, for every 0.12 ng/ml increase in serum dsDNA, the risk of occurrence of psoriasis OR was 4.88 (95% Cl: 3.85-6.20); when serum dsDNA≥ 1.11 ng/ml, for every one standard deviation 0.34 ng/ml increase in serum dsDNA, the risk of occurrence of psoriasis OR was 1.97 (95% Cl: 1.74-2.22) (Figure 11).
(3) In different subgroups of psoriasis (different regions, latitudes, temperatures, seasons, ultraviolets, humidities, sunlight exposure durations and daylight durations), and different cutoff values of serum dsDNA, serum dsDNA was positively correlated with the risk of occurrence of psoriasis (Figure 12-18). Meta-analysis of the results of all regions showed that without adjusting for confounding factors, for every one standard deviation increase in serum dsDNA, the psoriasis risk increased by 3.62 times (OR: 4.62, 95% Cl: 2.64-8.11); after adjusting for confounding factors, for every one standard deviation increase in serum dsDNA, the psoriasis risk increased by 1.84 times (OR: 2.84, 95% Cl: 2.01-4.01) (Figure 19).

### Example 5 Positive correlation between serum dsDNA and psoriasis severity (PASI and BSA score)

(1) In psoriasis patients, for every 1 ng/ml increase in serum dsDNA, the PASI score increased by 0.26 points, and the BSA score increased by 0.30 points (Table 8).

**Table 8 Linear correlation between dsDNA level and PASI score and BSA score in multicenter patients**

| | | PASI value* | | | BSA value* | | |
|---|---|---|---|---|---|---|---|
| | | *β* (95% Cl) | *t* | *P* | *β* (95% Cl) | *t* | *P* |
| All | Model 1 † | 0.66 (0.50 - | 8.001 | < 0.001 | 0.74 (0.50 - | 6.003 | < 0.001 |
| participants | | 0.82) | | | 0.98) | | |
| | Model 2 ‡ | 0.66 (0.50 - 0.82) | 8.085 | < 0.001 | 0.77 (0.53 - 1.01) | 6.281 | < 0.001 |
| | Model 3 § | 0.51 (0.36 ~ 0.66) | 6.629 | < 0.001 | 0.56 (0.32 - 0.79) | 4.697 | < 0.001 |
| | Model 4 # | 0.51 (0.36 ~ 0.67) | 6.675 | < 0.001 | 0.55 (0.32 - 0.78) | 4.615 | < 0.001 |
| Abbreviation: dsDNA, double-stranded DNA | | | | | | | |
| * Square root transformation on PASI score and BSA score was performed. | | | | | | | |
| † Model 1 had no variables adjusted. | | | | | | | |
| ‡ Model 2 was adjusted according to age, gender, and body mass index. | | | | | | | |
| § Model 3 was adjusted according to age, gender, body mass index, season, region, temperature, ultraviolet index, latitude, humidity, sunlight exposure duration, and daylight duration. | | | | | | | |
| # Model 4 was adjusted according to age, gender, body mass index, season, region, temperature, ultraviolet index, latitude, humidity, sunlight exposure duration, daylight duration, smoking, alcohol use, family history, complications, systemic medication, and topical medication. | | | | | | | |

(2) In most subgroups of region, latitude, temperature, season, ultraviolet intensity, humidity, sunlight exposure duration and daylight duration, serum dsDNA was significantly positively correlated with PASI and BSA scores. (Table 9)

**Table 9 Multicenter, subgroup analysis of the linear correlation between dsDNA level and PASI score and BSA score in patients.**

| Variables | Subgroups | PASI value* | | | BSA value* | | |
|---|---|---|---|---|---|---|---|
| | | *β* (95% Cl) | *t* | *P* | *β* (95% Cl) | *t* | *P* |
| Temperature | High | | | | | | |
| | Model 1† | 0.82 (0.58 - 1.06) | 6.692 | < 0.001 | 1.31 (0.95 - 1.67) | 7.106 | < 0.001 |
| | Model 2‡ | 0.78 (0.56 - 1.00) | 6.984 | < 0.001 | 1.42 (1.06 ~ 1.79) | 7.624 | < 0.001 |
| | Low | | | | | | |
| | Model 1† | 0.48 (0.26 ~ 0.70) | 4.232 | < 0.001 | 0.18 (-0.15 ~ 0.51) | 1.064 | 0.287 |
| | Model 2‡ | 0.44 (0.22 ~ 0.65) | 3.938 | < 0.001 | 0.22 (-0.11 ~ 0.55) | 1.324 | 0.186 |
| Season | Summer | | | | | | |
| | Model 1† | 1.37 (1.07 ~ 1.68) | 8.946 | < 0.001 | 1.90 (1.45 ~ 2.36) | 8.235 | < 0.001 |
| | Model 2‡ | 1.15 (0.88 ~ 1.42) | 8.267 | < 0.001 | 1.62 (1.17 ~ 2.06) | 7.162 | < 0.001 |
| | Spring and autumn | | | | | | |
| | Model 1† | -0.06(-0.41 ~ 0.29) | -0.343 | 0.732 | 0.46 (-0.06 ~ 0.99) | 1.726 | 0.085 |
| | Model 2‡ | -0.01 (-0.33 ~ 0.32) | -0.018 | 0.986 | 0.69 (0.18 ~ 1.20) | 2.668 | 0.008 |
| | Winter | | | | | | |
| | Model 1† | 0.51 (0.28 ~ 0.75) | 4.314 | < 0.001 | 0.03 (-0.32 ~ 0.38) | 0.161 | 0.872 |
| | Model 2‡ | 0.48 (0.26 ~ 0.69) | 4.342 | < 0.001 | 0.12 (-0.20 ~ 0.45) | 0.761 | 0.447 |
| Region | South China | | | | | | |
| | Model 1† | 0.26 (0.03 ~ 0.49) | 2.269 | 0.023 | 0.56 (0.22 ~ 0.91) | 3.220 | 0.001 |
| | Model 2‡ | 0.41 (0.19 ~ 0.63) | 3.695 | < 0.001 | 0.82 (0.49 ~ 1.16) | 4.843 | < 0.001 |
| | North China | | | | | | |
| | Model 1† | 0.59 (0.36 ~ 0.82) | 5.030 | < 0.001 | 0.59 (0.21 ~ 0.96) | 3.077 | 0.002 |
| | Model 2‡ | 0.55 (0.35 ~ 0.74) | 5.553 | < 0.001 | 0.30 (-0.01 ~ 0.62) | 1.904 | 0.057 |
| Daylight duration | Short | | | | | | |
| | Model 1† | 0.28 (-0.01 ~ 0.56) | 1.882 | 0.060 | 0.20 (-0.24 ~ 0.64) | 0.907 | 0.365 |
| | Model 2‡ | 0.20 (-0.03 ~ 0.44) | 1.707 | 0.088 | 0.22 (-0.14 ~ 0.58) | 1.205 | 0.229 |
| | Medium | | | | | | |
| | Model 1† | 0.89 (0.63 ~ 1.15) | 6.748 | < 0.001 | 0.65 (0.27 ~ 1.03) | 3.337 | 0.001 |
| | Model 2‡ | 0.71 (0.42 ~ 1.00) | 4.824 | < 0.001 | 0.78 (0.33 ~ 1.22) | 3.408 | 0.001 |
| | Long | | | | | | |
| | Model 1† | 0.75 (0.45 - 1.05) | 4.882 | < 0.001 | 1.47 (1.03 ~ 1.90) | 6.634 | < 0.001 |
| | Model 2‡ | 0.68 (0.42 ~ 0.94) | 5.103 | < 0.001 | 1.23 (0.77 ~ 1.69) | 5.273 | < 0.001 |
| Sunlight exposure duration | Short | | | | | | |
| | Model 1† | 0.39 (-0.75 ~ -0.03) | -2.152 | 0.032 | -0.27 (-0.81 ~ 0.27) | -0.972 | 0.331 |
| | Model 2‡ | -0.25 (0.60 ~ 0.10) | -1.391 | 0.165 | -0.04 (-0.59 ~ 0.51) | -0.151 | 0.880 |
| | Medium | | | | | | |
| | Model 1† | 0.43 (0.17 ~ 0.70) | 3.265 | 0.001 | 0.29 (-0.13 ~ 0.71) | 1.354 | 0.176 |
| | Model 2‡ | 0.35 (0.12 ~ 0.59) | 3.003 | 0.003 | 0.19 (-0.21 ~ 0.60) | 0.932 | 0.352 |
| | Long | | | | | | |
| | Model 1† | 0.91 (0.67 ~ 1.14) | 7.511 | < 0.001 | 1.21 (0.86 ~ 1.55) | 6.921 | < 0.001 |
| | Model 2‡ | 0.82 (0.58 ~ 1.06) | 6.721 | < 0.001 | 1.28 (0.93 ~ 1.63) | 7.178 | < 0.001 |
| Ultraviolet intensity | Medium and low | | | | | | |
| | Model 1† | 0.70 (0.49 ~ 0.91) | 6.570 | < 0.001 | 0.40 (0.09 ~ 0.71) | 2.530 | 0.012 |
| | Model 2‡ | 0.28 (0.07 ~ 0.48) | 2.673 | 0.008 | -0.03 (-0.33 ~ 0.27) | -0.204 | 0.838 |
| | High | | | | | | |
| | Model 1† | 0.71 (0.44 ~ 0.97) | 5.281 | < 0.001 | 1.36 (0.96 ~ 1.76) | 6.704 | < 0.001 |
| | Model 2‡ | 0.65 (0.41 ~ 0.89) | 5.244 | < 0.001 | 1.47 (1.08 ~ 1.87) | 7.319 | < 0.001 |
| Humidity | Low | | | | | | |
| | Model 1† | 0.63 (0.42 - 0.84) | 5.957 | < 0.001 | 0.52 (0.20 ~ 0.83) | 3.216 | 0.001 |
| | Model 2‡ | 0.44 (0.25 ~ 0.63) | 4.552 | < 0.001 | 0.32 (0.34 ~ 0.61) | 2.192 | 0.029 |
| | High | | | | | | |
| | Model 1† | 0.86 (0.59 ~ 1.14) | 6.185 | < 0.001 | 1.36 (0.96 ~ 1.77) | 6.592 | < 0.001 |
| | Model 2‡ | 0.65 (0.40 ~ 0.91) | 5.004 | < 0.001 | 1.48 (1.07 ~ 1.89) | 7.081 | < 0.001 |
| Latitude | Low | | | | | | |
| | Model 1† | 0.08 (-0.34 ~ 0.50) | 0.379 | 0.705 | 0.66 (0.00 ~ 1.33) | 1.964 | 0.050 |
| | Model 2‡ | 0.10 (-0.30 ~ 0.50) | 0.481 | 0.631 | 0.59 (-0.05 ~ 1.23) | 1.806 | 0.072 |
| | Middle | | | | | | |
| | Model 1† | 0.77 (0.60 ~ 0.95) | 8.535 | < 0.001 | 0.89 (0.62 ~ 1.15) | 6.568 | < 0.001 |
| | Model 2‡ | 0.65 (0.49 ~ 0.80) | 8.153 | < 0.001 | 0.67 (0.42 ~ 0.92) | 5.336 | < 0.001 |
| Abbreviation: dsDNA, double-stranded DNA. | | | | | | | |
| * Square root transformation on PASI score and BSA score was performed. | | | | | | | |
| † The model had no variables adjusted. | | | | | | | |
| ‡ The model had age, gender, season, body mass index, region, temperature, ultraviolet index, humidity, sunlight exposure duration, daylight duration, smoking, alcohol use, family medical history, complications, systemic medication, and topical medication adjusted. (The variables in this subgroup were not adjusted) | | | | | | | |

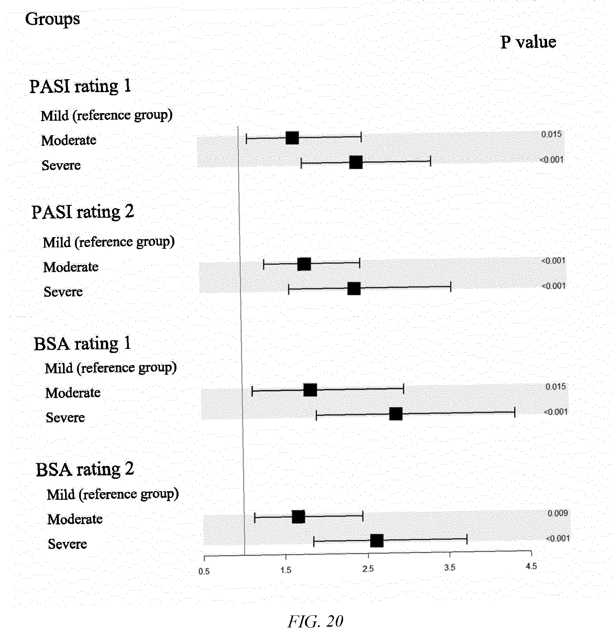

(3) The serum dsDNA level was also significantly correlated to the grade of psoriasis: for every 1 ng/ml increase in serum dsDNA, for psoriasis patients, the risk of being rated as moderate by PASI-1 increased by 0.66 times (OR: 1.66, 95% Cl: 1.10-2.50), and the risk of being rated as severe by PASI-1 increased by 1.43 times (OR: 2.43, 95% Cl: 1.77-3.35); the risk of being rated as moderate by PASI-2 increased by 0.78 times (OR: 1.78, 95% Cl: 1.29-2.46), and the risk of being rated as severe by PASI-2 increased by 1.38 times (OR: 2.38, 95% Cl: 1.59-3.57); the risk of being rated as moderate by BSA-1 increased by 0.83 times (OR: 1.83, 95% Cl: 1.12-2.97), and the risk of being rated as severe by BSA-1 increased by 1.87 times (OR: 2.87, 95% Cl: 1.90-4.33); the risk of being rated as moderate by BSA-2 increased by 0.66 times (OR: 1.66, 95% Cl: 1.13-2.45), and the risk of being rated as severe by BSA-2 increased by 1.61 times (OR: 2.61, 95% Cl: 1.84 -3.72) (Figure 20, Table 10).

**Table 10 The relationship between dsDNA value and the risk according to PASI score and BSA score in patients in the case of multicenter**

| → | | | | | |
|---|---|---|---|---|---|
| | | Model 1† | | Model 2‡ | |
| | | OR (95% Cl) | *P* | OR (95% Cl) | *P* |
| PASI grade 1 | | | | | |
| | Mild | Parameters | | Parameters | |
| | Moderate | 1.59 (1.09-2.33) | 0.017 | 1.56 (1.06-2.31) | 0.014 |
| | Severe | 2.33 (1.78-3.06) | < 0.001 | 2.44 (1.84-3.23) | < 0.001 |
| PASI grade 2 | | | | | |
| | Mild | Parameters | | Parameters | |
| | Moderate | 1.68 (1.27-2.24) | < 0.001 | 1.78 (1.33-2.38) | < 0.001 |
| | Severe | 2.58 (1.88-3.55) | < 0.001 | 2.65 (1.90-3.68) | < 0.001 |
| BSA grade 1 | | | | | |
| | Mild | Parameters | | Parameters | |
| | Moderate | 1.75 (1.14-2.69) | 0.011 | 1.72 (1.11-2.67) | 0.015 |
| | Severe | 2.96 (2.05-4.28) | < 0.001 | 2.82 (1.93-4.11) | < 0.001 |
| BSA grade 2 | | | | | |
| | Mild | Parameters | | Parameters | |
| | Moderate | 1.50 (1.07-2.11) | 0.018 | 1.61 (1.14-2.28) | 0.007 |
| | Severe | 1.99 (1.47-2.70) | < 0.001 | 2.06 (1.50-2.82) | < 0.001 |
| | | | | | |
| → | | | | | |
| | | Model 3§ | | Model 4# | |
| | | OR (95% Cl) | *P* | OR (95% Cl) | *P* |
| PASI | grade 1 | | | | |
| | Mild | Parameters | | Parameters | |
| | Moderate | 1.64 (1.09-2.45) | 0.017 | 1.66 (1.10-2.50) | 0.015 |
| | Severe | 2.28 (1.67-3.13) | < 0.001 | 2.43 (1.77-3.35) | < 0.001 |
| PASI grade 2 | | | | | |
| | Mild | Parameters | | Parameters | |
| | Moderate | 1.72 (1.25-2.36) | 0.001 | 1.78 (1.29-2.46) | < 0.001 |
| | Severe | 2.26 (1.52-3.37) | < 0.001 | 2.38 (1.59-3.57) | < 0.001 |
| BSA grade 1 | | | | | |
| | Mild | Parameters | | Parameters | |
| | Moderate | 1.79 (1.11-2.89) | 0.017 | 1.83 (1.12-2.97) | 0.015 |
| | Severe | 2.82 (1.88-4.24) | < 0.001 | 2.87 (1.90-4.33) | < 0.001 |
| BSA grade 2 | | | | | |
| | Mild | Parameters | | Parameters | |
| | Moderate | 1.64 (1.12-2.39) | 0.011 | 1.66 (1.13-2.45) | 0.009 |
| | Severe | 2.57 (1.82-3.64) | < 0.001 | 2.61 (1.84-3.72) | < 0.001 |
| Abbreviation: dsDNA, double-stranded DNA; OR, odds ratio; Cl: confidence interval. | | | | | |
| † Model 1 had no variables adjusted. | | | | | |
| ‡ Model 2 had age, gender, and body mass index adjusted. | | | | | |
| § Model 3 was adjusted according to age, gender, body mass index, season, region, temperature, ultraviolet degree, latitude, humidity, sunlight exposure duration, and daylight duration. | | | | | |
| # Model 4 was adjusted according to age, gender, body mass index, season, region, temperature, ultraviolet degree, latitude, humidity, sunlight exposure duration, daylight duration, smoking, alcohol use, family medical history, complications, systemic medication, and topical medication. | | | | | |

(4) Subgroup analysis showed that serum dsDNA levels were found to be significantly correlated with psoriasis grades in mid-latitude, high and low temperature, high and low ultraviolet, summer and winter, medium and long sunlight exposure duration, medium and long daylight duration, and north China subgroups. (Table 11).

**Table 11 Multicenter, subgroup analysis between dsDNA and the risk according to PASI score and BSA score in patients**

| Varia bles | Subgr oups | Psoriasis grades | | Original OR | 95% Cl | P | Correc ted OR | 95% Cl | *P* |
|---|---|---|---|---|---|---|---|---|---|
| Latit ude | Low | PASI grade 1 | | | | | | | |
| | | | Mild | Parame ters | | | Param eters | | |
| | | | Moderate | 1.99 | 0.70-5.63 | 0.197 | 1.37 | 0.43-4.34 | 0.591 |
| | | | Severe | 1.46 | 0.59-3.60 | 0.406 | 0.98 | 0.34-2.80 | 0.964 |
| | | PASI grade 2 | | | | | | | |
| | | | Mild | Parame ters | | | Param eters | | |
| | | | Moderate | 1.31 | 0.61-2.84 | 0.488 | 0.97 | 0.40-2.34 | 0.940 |
| | | | Severe | 0.03 | 0.01-0.48 | 0.013 | 0.06 | 0.01-2.89 | 0.153 |
| | | BSA grade 1 | | | | | | | |
| | | | Mild | Parame ters | | | Param eters | | |
| | | | Moderate | 1.29 | 0.13-12.42 | 0.823 | 3.46 | 0.15-79.52 | 0.438 |
| | | | Severe | 0.84 | 0.01-8.74 | 0.883 | 21.46 | 0.12-3806.57 | 0.246 |
| | | BSA grade 2 | | | | | | | |
| | | | Mild | Parame ters | | | Param eters | | |
| | | | Moderate | 0.77 | 0.23-2.63 | 0.682 | 0.48 | 0.13-1.77 | 0.269 |
| | | | Severe | 1.39 | 0.45-4.26 | 0.565 | 0.96 | 0.29-3.21 | 0.944 |
| | Middle | PASI grade 1 | | | | | | | |
| | | | Mild | Parame ters | | | Param eters | | |
| | | | Moderate | 1.96 | 1.27-3.01 | 0.002 | 1.62 | 1.04-2.55 | 0.035 |
| | | | Severe | 2.94 | 2.19-3.95 | < 0.001 | 2.81 | 1.99-3.96 | < 0.001 |
| | | PASI grade 2 | | | | | | | |
| | | | Mild | Parame ters | | | Param eters | | |
| | | | Moderate | 2.38 | 1.72-3.28 | < 0.001 | 1.98 | 1.40-2.81 | < 0.001 |
| | | | Severe | 2.63 | 1.89-3.66 | < 0.001 | 2.85 | 1.88-4.31 | < 0.001 |
| | | BSA grade 1 | | | | | | | |
| | | | Mild | Parame ters | | | Param eters | | |
| | | | Moderate | 2.01 | 1.28-3.16 | 0.002 | 1.76 | 1.07-2.91 | 0.026 |
| | | | Severe | 3.22 | 2.21-4.69 | < 0.001 | 2.84 | 1.87-4.30 | < 0.001 |
| | | BSA grade 2 | | | | | | | |
| | | | Mild | Parame ters | | | Param eters | | |
| | | | Moderate | 2.01 | 1.40-2.89 | < 0.001 | 1.83 | 1.22-2.74 | 0.003 |
| | | | Severe | 2.76 | 1.98-3.84 | < 0.001 | 2.65 | 1.82-3.86 | < 0.001 |

| Tem perat ure | High | PASI grade 1 | | | | | | | |
|---|---|---|---|---|---|---|---|---|---|
| | | | Mild | Parame ters | | | Param eters | | |
| | | | Moderate | 1.04 | 0.56-1.91 | 0.908 | 1.13 | 0.62-2.07 | 0.690 |
| | | | Severe | 2.57 | 1.77-3.73 | < 0.001 | 2.84 | 1.85-4.35 | < 0.001 |
| | | PASI grade 2 | | | | | | | |
| | | | Mild | Parame ters | | | Param eters | | |
| | | | Moderate | 1.84 | 1.22-2.76 | 0.004 | 2.05 | 1.32-3.18 | 0.001 |
| | | | Severe | 2.94 | 1.94-4.44 | < 0.001 | 3.01 | 1.80-5.05 | < 0.001 |
| | | BSA grade 1 | | | | | | | |
| | | | Mild | Parame ters | | | Param eters | | |
| | | | Moderate | 1.49 | 0.78-2.84 | 0.224 | 1.83 | 0.87-3.85 | 0.112 |
| | | | Severe | 3.69 | 2.15-6.33 | < 0.001 | 3.96 | 2.15-7.29 | < 0.001 |
| | | BSA grade 2 | | | | | | | |
| | | | Mild | Parame ters | | | Param eters | | |
| | | | Moderate | 0.95 | 0.54-1.66 | 0.858 | 1.26 | 0.68-2.32 | 0.467 |
| | | | Severe | 3.25 | 2.09-5.06 | < 0.001 | 4.92 | 2.95-8.21 | < 0.001 |
| | Low | PASI grade 1 | | | | | | | |
| | | | Mild | Parame ters | | | Param eters | | |
| | | | Moderate | 2.06 | 1.22-3.47 | 0.006 | 2.89 | 1.55-5.37 | 0.001 |
| | | | Severe | 2.30 | 1.52-3.47 | < 0.001 | 2.59 | 1.52-4.42 | < 0.001 |
| | | PASI grade 2 | | | | | | | |
| | | | Mild | Parame ters | | | Param eters | | |
| | | | Moderate | 1.59 | 1.05-2.39 | 0.027 | 1.98 | 1.19-3.30 | 0.009 |
| | | | Severe | 3.05 | 1.78-5.21 | < 0.001 | 1.72 | 0.84-3.51 | 0.138 |
| | | BSA grade 1 | | | | | | | |
| | | | Mild | Parame ters | | | Param eters | | |
| | | | Moderate | 1.99 | 1.09-3.64 | 0.025 | 1.77 | 0.88-3.58 | 0.112 |
| | | | Severe | 2.36 | 1.40-3.97 | 0.001 | 2.14 | 1.16-3.95 | 0.015 |
| | | BSA grade 2 | | | | | | | |
| | | | Mild | Parame ters | | | Param eters | | |
| | | | Moderate | 1.83 | 1.17-2.88 | 0.009 | 2.34 | 1.36-4.03 | 0.002 |
| | | | Severe | 1.17 | 0.74-1.84 | 0.494 | 1.32 | 0.76-2.30 | 0.319 |

| Ultra violet inten sity | Mediu m and low | PASI grade 1 | | | | | | | |
|---|---|---|---|---|---|---|---|---|---|
| | | | Mild | Parame ters | | | Param eters | | |
| | | | Moderate | 2.57 | 1.57-4.22 | < 0.001 | 2.92 | 1.61-5.31 | 0.001 |
| | | | Severe | 3.17 | 2.15-4.69 | < 0.001 | 2.51 | 1.53-4.14 | < 0.001 |
| | | PASI grade 2 | | | | | | | |
| | | | Mild | Parame ters | | | Param eters | | |
| | | | Moderate | 2.06 | 1.40-3.03 | < 0.001 | 1.85 | 1.14-3.01 | 0.013 |
| | | | Severe | 4.15 | 2.51-6.86 | < 0.001 | 2.26 | 1.17-4.36 | 0.015 |
| | | BSA grade 1 | | | | | | | |
| | | | Mild | Parame ters | | | Param eters | | |
| | | | Moderate | 2.39 | 1.37-4.16 | 0.002 | 1.82 | 0.95-3.47 | 0.069 |
| | | | Severe | 3.43 | 2.11-5.55 | < 0.001 | 2.18 | 1.25-3.82 | 0.006 |
| | | BSA grade 2 | | | | | | | |
| | | | Mild | Parame ters | | | Param eters | | |
| | | | Moderate | 2.20 | 1.44-3.35 | < 0.001 | 2.18 | 1.31-3.65 | 0.003 |
| | | | Severe | 1.56 | 1.03-2.38 | 0.036 | 1.25 | 0.75-2.08 | 0.392 |
| | High | PASI grade 1 | | | | | | | |
| | | | Mild | Parame ters | | | Param eters | | |
| | | | Moderate | 0.83 | 0.43-1.61 | 0.585 | 0.91 | 0.47-1.76 | 0.789 |
| | | | Severe | 2.32 | 1.54-3.48 | < 0.001 | 2.42 | 1.52-3.86 | < 0.001 |
| | | PASI grade 2 | | | | | | | |
| | | | Mild | Parame ters | | | Param eters | | |
| | | | Moderate | 1.61 | 1.03-2.52 | 0.037 | 1.75 | 1.09-2.81 | 0.020 |
| | | | Severe | 2.86 | 1.82-4.47 | < 0.001 | 2.58 | 1.47-4.52 | 0.001 |
| | | BSA grade 1 | | | | | | | |
| | | | Mild | Parame ters | | | Param eters | | |
| | | | Moderate | 1.32 | 0.62-2.78 | 0.469 | 1.57 | 0.68-3.61 | 0.284 |
| | | | Severe | 3.23 | 1.73-6.03 | < 0.001 | 3.51 | 1.77-6.96 | < 0.001 |
| | | BSA grade 2 | | | | | | | |
| | | | Mild | Parame | | | Param | | |
| | | | | ters | | | eters | | |
| | | | Moderate | 0.77 | 0.41-1.47 | 0.434 | 1.02 | 0.52-2.01 | 0.949 |
| | | | Severe | 3.50 | 2.10-5.83 | < 0.001 | 5.02 | 2.84-8.86 | < 0.001 |

| Seas on | Summ er | PASI grade 1 | | | | | | | |
|---|---|---|---|---|---|---|---|---|---|
| | | | Mild | Parame ters | | | Param eters | | |
| | | | Moderate | 2.07 | 0.94-4.54 | 0.070 | 2.51 | 1.13-5.55 | 0.024 |
| | | | Severe | 5.89 | 3.50-9.91 | < 0.001 | 5.46 | 3.09-9.67 | < 0.001 |
| | | PASI grade 2 | | | | | | | |
| | | | Mild | Parame ters | | | Param eters | | |
| | | | Moderate | 3.31 | 1.91-5.73 | < 0.001 | 3.56 | 1.99-6.33 | < 0.001 |
| | | | Severe | 7.14 | 4.05-12.59 | < 0.001 | 5.78 | 2.97-11.24 | < 0.001 |
| | | BSA grade 1 | | | | | | | |
| | | | Mild | Parame ters | | | Param eters | | |
| | | | Moderate | 1.23 | 0.51-2.94 | 0.643 | 1.12 | 0.42-3.01 | 0.819 |
| | | | Severe | 6.09 | 2.97-12.49 | < 0.001 | 5.10 | 2.35-11.05 | < 0.001 |
| | | BSA grade 2 | | | | | | | |
| | | | Mild | Parame ters | | | Param eters | | |
| | | | Moderate | 1.14 | 0.53-2.47 | 0.735 | 1.40 | 0.62-3.17 | 0.425 |
| | | | Severe | 7.20 | 3.86-13.43 | < 0.001 | 9.29 | 4.68-18.42 | < 0.001 |

| | Spring and autum n | PASI grade 1 | | | | | | | |
|---|---|---|---|---|---|---|---|---|---|
| | | | Mild | Parame ters | | | Param eters | | |
| | | | Moderate | 0.70 | 0.33-1.49 | 0.356 | 0.66 | 0.30-1.45 | 0.302 |
| | | | Severe | 1.02 | 0.62-1.67 | 0.944 | 1.17 | 0.61-2.25 | 0.631 |
| | | PASI grade 2 | | | | | | | |
| | | | Mild | Parame ters | | | Param eters | | |
| | | | Moderate | 1.01 | 0.58-1.75 | 0.966 | 0.99 | 0.52-1.90 | 0.981 |
| | | | Severe | 0.93 | 0.50-1.73 | 0.828 | 0.99 | 0.42-2.33 | 0.982 |
| | | BSA grade 1 | | | | | | | |
| | | | Mild | Parame ters | | | Param eters | | |
| | | | Moderate | 1.08 | 0.47-2.47 | 0.855 | 1.53 | 0.61-3.83 | 0.369 |
| | | | Severe | 1.29 | 0.65-2.55 | 0.465 | 1.93 | 0.89-4.22 | 0.097 |
| | | BSA grade 2 | | | | | | | |
| | | | Mild | Parame ters | | | Param eters | | |
| | | | Moderate | 0.92 | 0.46-1.86 | 0.821 | 1.09 | 0.51-2.32 | 0.818 |
| | | | Severe | 1.41 | 0.78-2.55 | 0.252 | 1.94 | 0.96-3.94 | 0.065 |
| | Winter | PASI grade 1 | | | | | | | |
| | | | Mild | Parame ters | | | Param eters | | |
| | | | Moderate | 2.03 | 1.13-3.65 | 0.018 | 2.50 | 1.23-5.08 | 0.011 |
| | | | Severe | 2.58 | 1.63-4.09 | < 0.001 | 1.93 | 1.04-3.58 | 0.036 |
| | | PASI grade 2 | | | | | | | |
| | | | Mild | Parame | | | Param | | |
| | | | | ters | | | eters | | |
| | | | Moderate | 1.56 | 0.99-2.47 | 0.056 | 1.59 | 0.88-2.88 | 0.126 |
| | | | Severe | 5.14 | 2.79-9.49 | < 0.001 | 1.98 | 0.86-4.55 | 0.107 |
| | | BSA grade 1 | | | | | | | |
| | | | Mild | Parame ters | | | Param eters | | |
| | | | Moderate | 2.90 | 1.47-5.71 | 0.002 | 2.30 | 1.03-5.12 | 0.041 |
| | | | Severe | 3.33 | 1.83-6.06 | < 0.001 | 2.06 | 1.01-4.17 | 0.046 |
| | | BSA grade 2 | | | | | | | |
| | | | Mild | Parame ters | | | Param eters | | |
| | | | Moderate | 1.81 | 1.11-2.96 | 0.017 | 2.05 | 1.11-3.76 | 0.021 |
| | | | Severe | 1.06 | 0.64-1.76 | 0.813 | 0.88 | 0.46-1.67 | 0.701 |

| Dayli ght durat ion | Short | PASI grade 1 | | | | | | | |
|---|---|---|---|---|---|---|---|---|---|
| | | | Mild | Parame ters | | | Param eters | | |
| | | | Moderate | 1.70 | 0.82-3.53 | 0.156 | 1.67 | 0.74-3.79 | 0.220 |
| | | | Severe | 0.80 | 0.42-1.52 | 0.489 | 0.86 | 0.39-1.89 | 0.713 |
| | | PASI grade 2 | | | | | | | |
| | | | Mild | Parame ters | | | Param eters | | |
| | | | Moderate | 0.77 | 0.42-1.41 | 0.400 | 0.86 | 0.41-1.80 | 0.691 |
| | | | Severe | 0.73 | 0.24-2.24 | 0.584 | 0.41 | 0.09-1.80 | 0.237 |
| | | BSA grade 1 | | | | | | | |
| | | | Mild | Parame ters | | | Param eters | | |
| | | | Moderate | 0.94 | 0.41-2.17 | 0.886 | 1.12 | 0.44-2.84 | 0.818 |
| | | | Severe | 0.79 | 0.38-1.62 | 0.517 | 1.02 | 0.44-2.34 | 0.969 |
| | | BSA grade 2 | | | | | | | |
| | | | Mild | Parame ters | | | Param eters | | |
| | | | Moderate | 0.82 | 0.41-1.64 | 0.580 | 1.14 | 0.52-2.46 | 0.746 |
| | | | Severe | 0.65 | 0.33-1.28 | 0.212 | 0.83 | 0.38-1.82 | 0.640 |

| | Mediu m | PASI grade 1 | | | | | | | |
|---|---|---|---|---|---|---|---|---|---|
| | | | Mild | Parame ters | | | Param eters | | |
| | | | Moderate | 2.05 | 1.13-3.71 | 0.017 | 2.44 | 1.16-5.15 | 0.019 |
| | | | Severe | 3.46 | 2.28-5.27 | < 0.001 | 3.50 | 1.99-6.15 | < 0.001 |
| | | PASI grade 2 | | | | | | | |
| | | | Mild | Parame ters | | | Param eters | | |
| | | | Moderate | 2.13 | 1.37-3.31 | 0.001 | 2.27 | 1.28-4.00 | 0.005 |
| | | | Severe | 3.77 | 2.33-6.10 | < 0.001 | 3.53 | 1.86-6.72 | < 0.001 |
| | | BSA grade 1 | | | | | | | |
| | | | Mild | Parame ters | | | Param eters | | |
| | | | Moderate | 2.70 | 1.38-5.32 | 0.004 | 3.08 | 1.29-7.35 | 0.011 |
| | | | Severe | 4.58 | 2.50-8.36 | < 0.001 | 3.41 | 1.59-7.31 | 0.002 |
| | | BSA grade 2 | | | | | | | |
| | | | Mild | Parame ters | | | Param eters | | |
| | | | Moderate | 2.41 | 1.46-3.96 | 0.001 | 2.25 | 1.18-4.28 | 0.014 |
| | | | Severe | 1.84 | 1.16-2.93 | 0.010 | 2.36 | 1.31-4.27 | 0.004 |
| | Long | PASI grade 1 | | | | | | | |
| | | | Mild | Parame ters | | | Param eters | | |
| | | | Moderate | 1.10 | 0.51-2.36 | 0.806 | 0.94 | 0.43-2.04 | 0.870 |
| | | | Severe | 2.63 | 1.63-4.22 | < 0.001 | 2.16 | 1.22-3.81 | 0.008 |
| | | PASI grade 2 | | | | | | | |
| | | | Mild | Parame ters | | | Param eters | | |
| | | | Moderate | 2.41 | 1.44-4.02 | 0.001 | 1.77 | 1.00-3.14 | 0.050 |
| | | | Severe | 2.68 | 1.60-4.50 | < 0.001 | 2.49 | 1.25-4.96 | 0.010 |
| | | BSA grade 1 | | | | | | | |
| | | | Mild | Parame ters | | | Param eters | | |
| | | | Moderate | 1.56 | 0.67-3.63 | 0.301 | 1.28 | 0.48-3.37 | 0.623 |
| | | | Severe | 4.43 | 2.23-8.78 | < 0.001 | 2.86 | 1.30-6.28 | 0.009 |
| | | BSA grade 2 | | | | | | | |
| | | | Mild | Parame ters | | | Param eters | | |
| | | | Moderate | 1.22 | 0.61-2.44 | 0.575 | 0.91 | 0.40-2.06 | 0.825 |
| | | | Severe | 4.66 | 2.61-8.34 | < 0.001 | 4.55 | 2.26-9.17 | < 0.001 |

| Sunli ght expo sure durat ion | Short | PASI grade 1 | | | | | | | |
|---|---|---|---|---|---|---|---|---|---|
| | | | Mild | Parame ters | | | Param eters | | |
| | | | Moderate | 1.66 | 0.74-3.73 | 0.218 | 1.57 | 0.62-4.00 | 0.341 |
| | | | Severe | 0.68 | 0.32-1.43 | 0.306 | 0.68 | 0.26-1.79 | 0.438 |
| | | PASI grade 2 | | | | | | | |
| | | | Mild | Paramee ters | | | Param eters | | |
| | | | Moderate | 0.75 | 0.38-1.46 | 0.395 | 0.69 | 0.29-1.64 | 0.405 |
| | | | Severe | 0.11 | 0.02-0.59 | 0.010 | 0.09 | 0.01-0.76 | 0.027 |
| | | BSA grade 1 | | | | | | | |
| | | | Mild | Parame ters | | | Param eters | | |
| | | | Moderate | 0.51 | 0.17-1.52 | 0.229 | 0.67 | 0.20-2.20 | 0.508 |
| | | | Severe | 0.48 | 0.18-1.28 | 0.145 | 0.69 | 0.21-2.24 | 0.537 |
| | | BSA grade 2 | | | | | | | |
| | | | Mild | Parame ters | | | Param eters | | |
| | | | Moderate | 0.98 | 0.42-2.27 | 0.962 | 1.08 | 0.41-2.82 | 0.879 |
| | | | Severe | 0.51 | 0.21-1.21 | 0.128 | 0.54 | 0.19-1.49 | 0.236 |

| | Mediu m | PASI grade 1 | | | | | | | |
|---|---|---|---|---|---|---|---|---|---|
| | | | Mild | Parame ters | | | Param eters | | |
| | | | Moderate | 1.41 | 0.73-2.71 | 0.304 | 1.83 | 0.84-4.02 | 0.130 |
| | | | Severe | 1.84 | 1.20-2.81 | 0.005 | 2.14 | 1.18-3.85 | 0.012 |
| | | PASI grade 2 | | | | | | | |
| | | | Mild | Parame ters | | | Param eters | | |
| | | | Moderate | 1.59 | 1.00-2.53 | 0.048 | 2.12 | 1.18-3.83 | 0.012 |
| | | | Severe | 1.88 | 1.18-3.00 | 0.008 | 2.21 | 1.12-4.37 | 0.023 |
| | | BSA grade 1 | | | | | | | |
| | | | Mild | Parame ters | | | Param eters | | |
| | | | Moderate | 3.40 | 1.64-7.03 | 0.001 | 4.62 | 1.81-11.79 | 0.001 |
| | | | Severe | 4.98 | 2.63-9.42 | < 0.001 | 3.92 | 1.74-8.86 | 0.001 |
| | | BSA grade 2 | | | | | | | |
| | | | Mild | Parame ters | | | Param eters | | |
| | | | Moderate | 1.97 | 1.17-3.32 | 0.011 | 2.12 | 1.08-4.16 | 0.030 |
| | | | Severe | 1.70 | 1.05-2.75 | 0.030 | 1.78 | 0.93-3.41 | 0.081 |
| | Long | PASI grade 1 | | | | | | | |
| | | | Mild | Parame ters | | | Param eters | | |
| | | | Moderate | 1.51 | 0.81-2.80 | 0.195 | 1.35 | 0.70-2.60 | 0.366 |
| | | | Severe | 2.91 | 1.90-4.47 | < 0.001 | 3.59 | 2.24-5.75 | < 0.001 |
| | | PASI grade 2 | | | | | | | |
| | | | Mild | Parame ters | | | Param eters | | |
| | | | Moderate | 2.08 | 1.32-3.27 | 0.002 | 2.30 | 1.40-3.77 | 0.001 |
| | | | Severe | 3.31 | 1.99-5.52 | < 0.001 | 4.27 | 2.41-7.56 | < 0.001 |
| | | BSA grade 1 | | | | | | | |
| | | | Mild | Parame ters | | | Param eters | | |
| | | | Moderate | 1.35 | 0.69-2.64 | 0.383 | 1.67 | 0.80-3.48 | 0.175 |
| | | | Severe | 2.98 | 1.74-5.12 | < 0.001 | 3.84 | 2.11-7.01 | < 0.001 |
| | | BSA grade 2 | | | | | | | |
| | | | Mild | Parame ters | | | Param eters | | |
| | | | Moderate | 1.14 | 0.63-2.08 | 0.659 | 1.31 | 0.69-2.50 | 0.412 |
| | | | Severe | 3.38 | 2.09-5.47 | < 0.001 | 4.76 | 2.80-8.11 | < 0.001 |

| Regi on | South China | PASI grade 1 | | | | | | | |
|---|---|---|---|---|---|---|---|---|---|
| | | | Mild | Parame ters | | | Param eters | | |
| | | | Moderate | 1.34 | 0.83-2.16 | 0.230 | 1.63 | 0.98-2.71 | 0.062 |
| | | | Severe | 1.49 | 1.02-2.19 | 0.041 | 2.33 | 1.51-3.61 | < 0.001 |
| | | PASI grade 2 | | | | | | | |
| | | | Mild | Parame ters | | | Param eters | | |
| | | | Moderate | 1.09 | 0.74-1.61 | 0.669 | 1.46 | 0.94-2.27 | 0.095 |
| | | | Severe | 2.21 | 1.30-3.76 | 0.003 | 2.67 | 1.46-4.88 | 0.001 |
| | | BSA grade 1 | | | | | | | |
| | | | Mild | Parame ters | | | Param eters | | |
| | | | Moderate | 0.81 | 0.45-1.44 | 0.474 | 1.04 | 0.55-1.95 | 0.902 |
| | | | Severe | 1.29 | 0.80-2.09 | 0.294 | 1.61 | 0.96-2.71 | 0.070 |
| | | BSA grade 2 | | | | | | | |
| | | | Mild | Parame ters | | | Param eters | | |
| | | | Moderate | 0.80 | 0.49-1.30 | 0.370 | 1.03 | 0.62-1.74 | 0.897 |
| | | | Severe | 1.53 | 1.01-2.32 | 0.044 | 2.40 | 1.52-3.79 | < 0.001 |

| | North China | PASI grade 1 | | | | | | | |
|---|---|---|---|---|---|---|---|---|---|
| | | | Mild | Parame ters | | | Param eters | | |
| | | | Moderate | 2.39 | 1.25-4.58 | 0.008 | 1.68 | 0.75-3.77 | 0.210 |
| | | | Severe | 2.34 | 1.55-3.54 | < 0.001 | 2.72 | 1.54-4.78 | 0.001 |
| | | PASI grade 2 | | | | | | | |
| | | | Mild | Parame ters | | | Param eters | | |
| | | | Moderate | 2.46 | 1.57-3.85 | < 0.001 | 2.90 | 1.63-5.14 | < 0.001 |
| | | | Severe | 1.72 | 1.11-2.66 | 0.016 | 2.23 | 1.17-4.26 | 0.015 |
| | | BSA grade 1 | | | | | | | |
| | | | Mild | Parame ters | | | Param eters | | |
| | | | Moderate | 6.02 | 3.08-12.49 | < 0.001 | 2.45 | 1.03-5.86 | 0.043 |
| | | | Severe | 9.10 | 4.97-16.65 | < 0.001 | 3.13 | 1.45-6.73 | 0.003 |
| | | BSA grade 2 | | | | | | | |
| | | | Mild | Parame ters | | | Param eters | | |
| | | | Moderate | 3.70 | 2.17-6.32 | < 0.001 | 2.43 | 1.22-4.83 | 0.011 |
| | | | Severe | 3.46 | 2.11-5.68 | < 0.001 | 2.23 | 1.15-4.31 | 0.017 |

| Humi dity | Low | PASI grade 1 | | | | | | | |
|---|---|---|---|---|---|---|---|---|---|
| | | | Mild | Parame ters | | | Param eters | | |
| | | | Moderate | 1.75 | 1.09-2.82 | 0.021 | 1.79 | 1.07-3.00 | 0.026 |
| | | | Severe | 2.94 | 2.06-4.20 | < 0.001 | 2.41 | 1.59-3.66 | < 0.001 |
| | | PASI grade 2 | | | | | | | |
| | | | Mild | Parame ters | | | Param eters | | |
| | | | Moderate | 2.16 | 1.49-3.12 | < 0.001 | 1.77 | 1.17-2.67 | 0.007 |
| | | | Severe | 2.94 | 1.90-4.54 | < 0.001 | 2.63 | 1.54-4.51 | < 0.001 |
| | | BSA grade 1 | | | | | | | |
| | | | Mild | Parame ters | | | Param eters | | |
| | | | Moderate | 2.02 | 1.22-3.36 | 0.006 | 1.52 | 0.86-2.69 | 0.153 |
| | | | Severe | 2.48 | 1.62-3.80 | < 0.001 | 1.92 | 1.19-3.09 | 0.007 |
| | | BSA grade 2 | | | | | | | |
| | | | Mild | Parame ters | | | Param eters | | |
| | | | Moderate | 2.01 | 1.35-3.01 | 0.001 | 1.81 | 1.14-2.87 | 0.011 |
| | | | Severe | 1.97 | 1.33-2.91 | 0.001 | 1.62 | 1.03-2.54 | 0.037 |

| | High | PASI grade 1 | | | | | | | |
|---|---|---|---|---|---|---|---|---|---|
| | | | Mild | Parame ters | | | Param eters | | |
| | | | Moderate | 1.78 | 0.90-3.52 | 0.097 | 1.39 | 0.67-2.91 | 0.379 |
| | | | Severe | 3.41 | 2.11-5.52 | < 0.001 | 2.34 | 1.34-4.09 | 0.003 |
| | | PASI grade 2 | | | | | | | |
| | | | Mild | Parame ters | | | Param eters | | |
| | | | Moderate | 2.03 | 1.24-3.32 | 0.005 | 1.70 | 0.98-2.94 | 0.060 |
| | | | Severe | 4.38 | 2.55-7.50 | < 0.001 | 2.64 | 1.32-5.27 | 0.006 |
| | | BSA grade 1 | | | | | | | |
| | | | Mild | Parame ters | | | Param eters | | |
| | | | Moderate | 2.18 | 0.91-5.23 | 0.079 | 1.81 | 0.64-5.07 | 0.261 |
| | | | Severe | 6.34 | 2.91-13.83 | < 0.001 | 5.78 | 2.27-14.72 | < 0.001 |
| | | BSA grade 2 | | | | | | | |
| | | | Mild | Parame ters | | | Param eters | | |
| | | | Moderate | 1.29 | 0.65-2.55 | 0.465 | 1.15 | 0.54-2.45 | 0.713 |
| | | | Severe | 4.43 | 2.45-8.01 | < 0.001 | 4.88 | 2.51-9.48 | < 0.001 |
| Abbreviation: dsDNA, double-stranded DNA; OR, odds ratio; Cl: confidence interval. The adjusted model had age, gender, body mass index, smoking, alcohol use, family history, topical medication, systemic medication, complications, season, region, latitude, humidity, temperature, ultraviolet index, daylight duration and sunlight exposure duration corrected (variables in this subgroup were not corrected). | | | | | | | | | |

Finally, it should be stated that the above examples are merely used for illustrating rather than limiting the technical solutions of the present invention. Although the present invention has been described in detail with reference to the preferred examples, a person skilled in the art should understand that modifications or equivalent substitutions may be made to the technical solutions of the present invention without departing from the spirit and scope of the technical solutions of the present invention, and all should be encompassed within the scope of the claims of the present invention.

## Claims

1. An evaluation system for evaluating psoriasis, wherein the evaluation system comprises:
a data acquisition module for acquiring the dsDNA content of a serum sample to be detected;
a data analysis module for evaluating psoriasis based on the dsDNA content;
a data output module for outputting results according to the evaluated psoriasis, wherein
if the serum sample to be detected is a serum sample without phenotype and the dsDNA content is ≥ 1.11 ng/ml, the output would be: abnormal and extremely high risk.

2. The evaluation system according to claim 1, wherein if the serum sample to be detected is a serum sample without phenotype and the dsDNA content is 0.97 ng/ml ≤ dsDNA content < 1.11 ng/ml, the output would be: normal and high risk; if the dsDNA content is 0.86 ng/ml ≤ dsDNA content < 0.97 ng/ml, the output would be: normal; or if the dsDNA content is 0.86 ng/ml, the output would be: suspicious of other diseases.

3. The evaluation system according to claim 1, wherein in the case that the serum sample to be detected is a serum sample without phenotype, the data acquisition module acquires the dsDNA content twice or more at different time points, for every one standard deviation increase in the dsDNA content at the later time point from the dsDNA content at the previous time point, the risk increases by 1.84 times (OR: 2.84, 95% Cl: 2.01-4.01), and the output module outputs whether the dsDNA content is normal and the corresponding risk factor.

4. The evaluation system according to claim 1, wherein in the case that the serum sample to be detected is a serum sample without phenotype, when the dsDNA content is < 1.11 ng/ml, for every 0.12 ng/ml increase in the difference between the dsDNA content at the later time point and the dsDNA content at the previous time point recorded by the data acquisition module, the OR value of the risk of occurrence of psoriasis is 4.88 (95% Cl: 3.85-6.20), and the output module outputs whether the dsDNA content is normal and the corresponding OR value; when the dsDNA content is ≥ 1.11 ng/ml, for every 0.34 ng/ml increase in the difference between the dsDNA content at the later time point and the dsDNA content at the previous time point recorded by the data acquisition module, the OR value of the risk of occurrence of psoriasis is 1.97 (95% Cl: 1.74-2.22), and the output module outputs whether the dsDNA content is normal and the corresponding OR value.

5. The evaluation system according to claim 1, wherein in the case that the serum sample to be detected is a phenotyped and cured serum sample, and the dsDNA content is ≥ 1.11 ng/m, then the output would be: very likely to relapse.

6. The evaluation system according to claim 1, wherein in the case that the serum sample to be detected is a phenotyped serum sample, the data acquisition module acquires the dsDNA content twice or more at different time points, the data analysis module uses a PASI method for evaluation, and the data output module outputs corresponding results; the PASI method is one of the following: (1) for the phenotyped serum sample, for every 1 ng/ml increase in the dsDNA content at the later time point from the dsDNA content at the previous time point, the risk of being rated as moderate by PASI-1 increases by 0.66 times (OR: 1.66, 95% Cl: 1.10-2.50), and the risk of being rated as severe by PASI-1 increases by 1.43 times (OR: 2.43, 95% Cl: 1.77-3.35); or (2) for the phenotyped serum sample, for every 1 ng/ml increase in the dsDNA content at the later time point from the dsDNA content at the previous time point, the risk of being rated as moderate by PASI-2 increases by 0.78 times (OR: 1.78, 95% Cl: 1.29-2.46), and the risk of being rated as severe by PASI-2 increases by 1.38 times (OR: 2.38, 95% Cl: 1.59-3.57).

7. The evaluation system according to claim 1, wherein in the case that the serum sample to be detected is a phenotyped serum sample, the data acquisition module acquires the dsDNA content twice or more at different time points, the data analysis module uses a BSA method for evaluation, and the data output module outputs corresponding results; the BSA method is one of the following: (1) for the phenotyped serum sample, for every 1 ng/ml increase in the dsDNA content at the later time point from the dsDNA content at the previous time point, the risk of being rated as moderate by BSA-1 increases by 0.83 times (OR: 1.83, 95% Cl: 1.12-2.97), and the risk of being rated as severe by BSA-1 increases by 1.87 times (OR: 2.87, 95% Cl: 1.90-4.33); or (2) for the phenotyped serum sample, for every 1 ng/ml increase in the dsDNA content at the later time point from the dsDNA content at the previous time point, the risk of being rated as moderate by BSA-2 increases by 0.66 times (OR: 1.66, 95% Cl: 1.13-2.45), and the risk of being rated as severe by BSA-2 increases by 1.61 times (OR: 2.61, 95% Cl: 1.84-3.72).

8. The evaluation system according to claim 1, wherein the evaluation system further comprises a case module for recording the dsDNA content of the serum sample to be detected at different time points and whether there is a history of psoriasis.

9. Use of a dsDNA binding agent in the preparation of a psoriasis detection reagent/kit.

10. An apparatus for the treatment of psoriasis, wherein the apparatus comprises an ultraviolet emitting device.

11. A high-throughput detection and analysis method for dsDNA, wherein the method comprises the following steps:
(1) detecting serum dsDNA content by using a double-stranded DNA quantitative detection method;
(2) calibrating the detection results with a standard curve and a 0 hole, and the data with the r2 value of the linear correlation relationship of the standard curve greater than 0.99 being retained;
(3) performing data processing and analysis by using SPSS and R Version.
